# EUROPÄISCHE PATENTANMELDUNG

(11) **EP 1 145 733 A2**
(43) Veröffentlichungstag der Anmeldung: **17.10.2001**
(21) Anmeldenummer: 01109191.5
(22) Anmeldetag: 12.04.2001
(51) Int. Cl.: A61N 1/36

(54) **Mindestens teilimplantierbares System zur Rehabilitation einer Hörstörung**

(30) Priorität: 13.04.2000 DE 10018360
(71) Anmelder: IMPLEX Aktiengesellschaft Hearing Technology, 85737 Ismaning (DE)
(72) Erfinder: Leysieffer, Hans, Dr.-Ing., 82024 Taufkirchen (DE); Zenner, Hans Peter, Prof. Dr.Dr.med., 72070 Tübingen (DE); Baumann, Joachim W., 85570 Markt Schwaben (DE)
(74) Vertreter: Schwan - Schwan - Schorer

(57) **Zusammenfassung**

Mindestens teilweise implantierbares System zur Rehabilitation einer Hörstörung, das mindestens einen Sensor (Mikrofon) (40) zur Aufnahme des Schallsignals und dessen Umwandlung in entsprechende elektrische Signale, eine elektronische Signalverarbeitungseinheit (34, 34') zur Audiosignalverarbeitung und -verstärkung, eine elektrische Energieversorgungseinheit (52), welche einzelne Komponenten des Systems mit Strom versorgt, sowie eine ausgangsseitige aktorische Stimulationsanordnung (10, 14, 21) umfasst. Erfindungsgemäß ist als aktorische Stimulationsanordnung eine duale intracochleäre Anordnung vorgesehen, die in Kombination eine Stimulatoranordnung mit mindestens einem Stimulatorelement (14 und/oder 21) zur mindestens mittelbaren mechanischen Stimulation des Innenohres und eine elektrisch wirkende Reizelektrodenanordnung mit mindestens einer Cochlea-Implant-Elektrode (11) zur elektrischen Stimulation des Innenohres aufweist.

## Beschreibung

Die vorliegende Erfindung betrifft ein mindestens teilweise implantierbares System zur Rehabilitation einer Hörstörung, das mindestens einen Sensor (Mikrofon) zur Aufnahme des Schallsignals und dessen Umwandlung in entsprechende elektrische Signale, eine elektronische Signalverarbeitungseinheit zur Audiosignalverarbeitung und -verstärkung, eine elektrische Energieversorgungseinheit, welche einzelne Komponenten des Systems mit Strom versorgt, sowie eine ausgangsseitige aktorische Stimulationsanordnung umfasst.

Unter dem Begriff "Hörstörung" sollen vorliegend Innenohrschäden, kombinierte Innen- und Mittelohrschäden sowie auch zeitweise auftretende oder permanente Ohrgeräusche (Tinnitus) verstanden werden.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea-Implantaten (CI) wird ein Array von Cochlea-Implant-Elektroden in die Cochlea eingeführt. Das von einem elektronischen System (Elektronikmodul) angesteuert wird. Dieses Elektronikmodul ist hermetisch dicht und biokompatibel eingekapselt, und es wird operativ im knöchernen Bereich hinter dem Ohr (Mastoid) eingebettet. Das elektronische System enthält jedoch im wesentlichen nur Dekodier- und Treiberschaltungen für die Reizelektroden. Die akustische Schallaufnahme, die Wandlung dieses Schallsignals in elektrische Signale und deren weitere Verarbeitung erfolgen grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO), und es ist mit einem Kabel mit dem Sprachprozessor verbunden. Solche Cochlea-Implantat-Systeme, deren Komponenten und Prinzipien der transkutanen Signalübertragung sind beispielhaft in folgenden Patentschriften beschrieben: US-A-5 070 535, US-A-4 441 210, EP-A-0 200 321, US-A-5 626 629. Verfahren zur Sprachaufbereitung und -Kodierung bei Cochleaimplantaten sind beispielsweise in folgenden Patentschriften angegeben: EP-A-0 823 188, EP-A-0 190 836, US-A-5 597 380, US-A-5 271 397, US-A-5 095 904, US-A-5 601 617, US-A-5 603 726.

Neben der Rehabilitation gehörloser beziehungsweise ertaubter Patienten mit Cochlea Implantaten existieren seit geraumer Zeit Ansätze, Patienten mit einer sensorineuralen Hörstörung, die operativ nicht behebbar ist, mit teil- beziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie zum Beispiel durch elektromagnetische und piezoelektrische Systeme. Der Vorteil dieser Geräte wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von Yanigahara und Suzuki et al. (Arch Otolaryngol Head Neck, Surg-Vol 113, 1987, pp. 869-872; Suzuki et al. "Implantation of Partially Implantable Middle Ear Implant and the Indication" Advances in Audiology, Vol. 4, 160-166, Karger Basel, 1988; H.P. Zenner et al. "Erste Implantationen eines vollständig implantierbaren elektronischen Hörsystems bei Patienten mit Innenohrschwerhörigkeit", HNO 46:844-852; H. Leysieffer et al. "Ein vollständig implantierbares Hörsystem für Innenohrschwerhörige: TICA LZ 3001" HNO 46:853-863; H.P. Zenner et al. "Aktive elektronische Hörimplantate für Mittel- und Innenohrschwerhörige - eine neue Ära der Ohrchirurgie", HNO 45:749-774; H.P. Zenner et al. "Totally implantable hearing device for sensorineural hearing loss", The Lancet Vol. 352, No. 9142, Seite 1751; und in zahlreichen Patentschriften beschrieben, so unter anderem in: EP-A-0 263 254, EP-A-0 400 630, EP-A-0 499 940, US-A-3 557 775, US-A-3 712 962, US-A-3 764 748, US-A-5 411 467, US-A-4 352 960, US-A-4 988 333, US-A-5 015 224, US-A-5 015 225, US-A-5 360 388, US-A-5 772 575, US-A-5 814 095, US-A-5 951 601, US-A-5 977 689 und US-A-5 984 859.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sogenannte Tinnitus-Maskierer bekannt (WO-A-90/07251, EP-A-0 537 385, DE-U-296 16 956). Dies sind kleine, batteriebetriebene Geräte, die ähnlich einem Hörgerät hinter oder im Ohr getragen werden und durch artifizielle Schalle, die beispielsweise über einen Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (zum Beispiel Terzrauschen), die in ihrer spektralen Lage und ihrem Lautstärkepegel über ein Programmiergerät einstellbar sind, um eine möglichst optimale Anpassung an die individuelle Ohrgeräuschsituation zu ermöglichen. Darüberhinaus existiert seit kurzem die sog. "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll (H. Knör, "Tinnitus-Retraining-Therapie und Hörakustik" Zeitschrift "Hörakustik" 2/97, Seiten 26 und 27). Diese Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In US-A-5 795 287 wird ein implantierbarer Tinnitusmaskierer mit "Direktantrieb" ("direct drive") des Mittelohres zum Beispiel über einen an die Ossikelkette angekoppelten elektromechanischen Wandler beschrieben. Dieser direkt gekoppelte Wandler kann vorzugsweise ein sogenannter "Floating Mass Transducer" (FMT) sein. Dieser FMT entspricht dem Wandler für implantierbare Hörgeräte, der in US-A-5 624 376 beschrieben ist.

In DE-C-198 58 398 und der DE-C- 198 59 171.3 werden implantierbare Systeme zur Behandlung eines Tinnitus durch Maskierung und/oder Noiserfunktionen beschrieben, bei denen der signalverarbeitende elektronische Pfad eines teil- oder vollimplantierbaren Hörsystems durch entsprechende elektronische Module so ergänzt wird, daß die zur Tinnitusmaskierung oder zur Noiserfunktion notwendigen Signale in den Signalverarbeitungsweg der Hörgerätefunktion eingespeist und die zugehörigen Signalparameter durch weitere elektronische Maßnahmen individuell an die pathologischen Bedürfnisse angepaßt werden können. Diese Anpassbarkeit kann dadurch realisiert werden, daß die notwendigen Einstelldaten der Signalerzeugungs- und Einspeiselektronik in demselben physikalischen und logischen Datenspeicherbereich des Implantatsystems hard- und softwaremäßig abgelegt beziehungsweise programmiert werden und über entsprechende elektronische Stellglieder die Einspeisung des Maskiererbeziehungsweise Noisersignals in den Audiopfad des Hörimplantats steuern.

Implantierbare Rehabilitationsgeräte der vorstehend genannten Art bestehen je nach angestrebter Funktion aus mehreren Funktionseinheiten, insbesondere einem Sensor (Mikrofon), der den einfallenden Luftschall in ein elektrisches Signal umwandelt, einer elektronischen Signalverarbeitungs-, Verstärkungs- und Implantatsteuerungseinheit, einem implantierbaren elektromechanischen beziehungsweise elektroakustischen Wandler, der die verstärkten und vorverarbeiteten Sensorsignale in mechanische beziehungsweise akustische Schwingungen umwandelt und diese über geeignete Koppelmechanismen dem geschädigten Mittel- und/oder Innenohr zuführt, oder einer cochleären Reizelektrode bei Cochlea-Implantaten, sowie einem elektrischen Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Besonders vorteilhafte Vorrichtungen und Verfahren zum Laden von nachladbaren Implantatbatterien sind in DE-A-198 38 137 sowie in EP-B-0 499 939 beschrieben. Zweckmäßig ist auch eine Telemetrieeinheit vorzusehen, mit der patientenspezifische, audiologische Daten drahtlos bidirektional übertragen beziehungsweise im Implantat programmiert und damit dauerhaft gespeichert werden können, wie es von Leysieffer et al. in HNO Vol. 46, 1998, S. 853-863 beschrieben wurde.

Grundsätzlich sind bei allen genannten, mindestens teilweise implantierbaren Systemen die (Audio-)Signalverarbeitung beziehungsweise Signalerzeugung sowie die Module der Implantatsteuerung wie z.B. ein geregeltes Akkumulator-Nachladesystem oder ein Telemetriesystem zur bidirektionalen Übertragung von z.B. veränderlichen, patientenspezifischen Parametern implantatseitig durch fest vorgegebene Hardwareeinheiten realisiert. Dies gilt auch dann, wenn zur Signalverarbeitung beziehungsweise -erzeugung oder zum Implantatmanagement digitale Signalprozessoren oder Microcontroller beziehungsweise Mikroprozessoren eingesetzt werden, unabhängig davon, ob diese als eine sogenannte "hardwired logic", das heißt in "festverdrahteter" Logikarchitektur, realisiert sind oder ob deren Betriebsprogramme in Festwertspeicherbereichen (z.B. ROM) der entsprechenden Prozessoren abgelegt sind. Diese Programme, die zum grundlegenden Betrieb des Implantats sowie zur bestimmungsgemäßen Funktion notwendig und vorgesehen sind, werden im folgenden als Betriebsprogramm beziehungsweise als Betriebssoftware bezeichnet. Diese Betriebssoftware wird bei den bekannten Implantatsystemen während der Produktion zum Beispiel durch Maskenprogrammierung der Prozessor-Speicherbereiche in das System eingebracht, und sie ist nach der Implantation nicht mehr veränderbar.

Im Gegensatz dazu werden patientenspezifische Daten wie z.B. audiologische Anpaßdaten oder auch veränderbare Implantatsystemparameter (z.B. als Variable in einem der oben genannten Softwareprogramme zur Regelung einer Akkumulatornachladung) vorliegend als Betriebsparameter bezeichnet. Diese Betriebsparameter können bei bekannten vollimplantierbaren Implantatsystemen nach der Implantation transkutan, das heißt, drahtlos durch die geschlossene Haut, in das Implantat übertragen und damit verändert werden.

Die oben beschriebenen mindestens teilweise implantierbaren Hörsysteme zur Rehabilitation einer Innenohrschädigung, die auf einem ausgangsseitigen elektromechanischen Wandler basieren, unterscheiden sich von herkömmlichen, konventionellen Hörgeräten im wesentlichen nur dadurch, daß der ausgangsseitige akustische Stimulus (das heißt ein verstärktes Schallsignal vor dem Trommelfell) durch einen verstärkten mechanischen Stimulus des Mittel- beziehungsweise Innenohres ersetzt wird. Der akustische Stimulus eines konventionellen Hörgerätes führt schließlich über die mechanische Anregung des Trommelfells und des sich anschließenden Mittelohres auch zu einem vibratorischen, das heißt mechanischen Reiz des Innenohres. Bezüglich der sinnvollen Audiosignalvorverarbeitung bestehen grundlegend ähnliche beziehungsweise gleiche Anforderungen. Weiterhin wird in beiden Ausführungsformen letztendlich ausgangsseitig ein örtlich lokalisierter vibratorischer Stimulus an das geschädigte Innenohr geleitet (zum Beispiel verstärkte mechanische Schwingung des Steigbügels im ovalen Fenster des Innenohres).

Grundsätzlich besteht bei diesen heute routinemäßig eingesetzten Rehabilitationen eines Innenohrschadens durch aktive Hörsysteme (gleich ob extern akustisch oder implantiert elektromechanisch) ein gravierender Nachteil, der im folgenden zum Verständnis der vorliegenden Erfindung zusammenfassend beschrieben wird: Die überwiegende Mehrheit der Innenohrschwerhörigkeiten basiert auf einer mehr oder weniger ausgeprägten Schädigung der äußeren Haarzellen im Innenohr. Diese äußeren Haarzellen, die in einer Vielzahl im Cortischen Organ entlang der Basilarmembran angeordnet sind, bilden einen Teil des sogenannten cochleären Verstärkers, der je nach örtlicher Anregung der Basilarmembran aufgrund einer Wanderwellenausbildung diesen örtlichen Anregungsbereich bei kleinen Pegeln und damit kleinen Wanderwellenamplituden mechanisch aktiv entdämpft und somit zu einer Empfindlichkeitssteigerung führt. Diese aktive Entdämpfung beruht auf einem sehr komplexen, efferent gesteuerten Prozeß, der hier nicht näher beschrieben wird. Es wird weiterhin angenommen, daß sich bei sehr hohen Pegeln der Innenohranregung aufgrund hoher Laustärke dieser Effekt in seiner Wirkung umkehrt und damit die Wanderwellenamplitude örtlich herarbsetzt und damit aktiv bedämpft. Diese nichtlineare Kennlinie des cochleären Verstärkers, der in mehreren hundert örtlich begrenzt wirkenden Funktionseinheiten entlang des Cortischen Organs angeordnet ist, ist für die Funktion des gesunden Innenohres von maßgeblicher Bedeutung. Bei teilweisem oder vollständigem Ausfall der äußeren Haarzellen entstehen neben dem Empfindlichkeitsverlust, der zu einer Hörschwellenanhebung führt, jedoch noch weitere Nachteile: die beschriebene aktive Entdämpfung der Basilarmembran führt zu hohen Güten der Einhüllenden der Wanderwellen, die wesentlich für das Frequenzunterscheidungsvermögen (Tonhöhenunterschiede) verantwortlich sind. Fehlt diese hohe Güte durch Ausfall oder Teilschädigung der äußeren Haarzellen, kann der Betroffene deutlich schlechter Tonhöhenunterschiede wahrnehmen. Die Anhebung der Hörschwelle führt darüberhinaus zu einer Verringerung des Dynamikbereiches, da sich die obere Empfindungsgrenze (Unbehaglichkeitsschwelle) bei einer Innenohrschwerhörigkeit nicht mit anhebt. Diese Reduktion der Dynamik hat eine Versteilerung des Lautstärkeempfindens zur Folge, was als positives Recruitment bezeichnet wird. Die beschriebenen Effekte, die durch Schädigung oder Ausfall der äußeren Haarzellen hervorgerufen werden, führen in der Gesamtwirkung für den Betroffenen zur Minderung der Sprachverständlichkeit insbesondere in störlärmerfüllter Umgebung (zusammenfassende Darstellung in Zenner, H.P.: "Hören", Georg Thieme Verlag Stuttgart, New York, 1994, Seiten 20-23, 107 und 108, und LePage, E.W., Johnstone, M.B.: "Non-linear mechanical behaviour of the basilar membrane in the basal turn of the guinea pig cochlea". Hearing Research 2 (1980), 183-189).

Wesentliche Konsequenz dieses beschriebenen Mechanismus ist, daß, wie oben angegeben, sowohl bei konventionellen, akustischen Hörgeräten wie auch bei teil- oder vollimplantierbaren Hörsystemen die wichtigen Funktionen der geschädigten äußeren Haarzellen und damit des cochleären Verstärkers nicht ersetzt oder zumindest teilweise wiederhergestellt werden können. Aus DE-C-198 40 211 ist eine Wandleranordnung für teil- oder vollimplantierbare Hörgeräte zur direkten mechanischen Anregung des Mittel- oder Innenohres bekannt, die mit einem piezoelektrischen Wandlerelement und zusätzlich mit einem elektromagnetischen Wandler versehen ist, die in einem gemeinsamen Gehäuse untergebracht sind und die beide über dasselbe Koppelelement mit ein Mittelohr-Ossikel oder direkt mit dem Innenohr koppelbar sind. Es sind ferner implantierbare Hörsysteme bekannt (WO-A-99/08476 entsprechend US-A-5 997 466, WO-A-99/08480 entsprechend US-A-6 005 955), die mit zwei oder mehr ausgangsseitigen elektromechanischen Wandlern in einer oder örtlich getrennten Anordnungen arbeiten; diese Ausführungsformen sind aber eindeutig dahingehend beschrieben, daß die Systemauslegung mit mehr als einem Wandler eine lineare Superposition der Auslenkungsfrequenzgängen der einzelnen Wandler ermöglicht, die im Ergebnis eine spektral möglichst optimierte beziehungsweise gezielt frequenzabhängig einstellbare oder programmierbare ausgangsseitige Anregungsform der Cochlea erlaubt und somit zu einem spektral ausgeglichenen und ausreichenden Lautstärkeeindruck des Implantatsystems führen soll. Eine Rehabilitation des cochleären Verstärkers mit den oben beschriebenen Merkmalen ist durch diese Ausführungsformen beziehungsweise beschriebenen Signalvorverarbeitungsmethoden jedoch nicht möglich.

Bei den Cochlea Implantaten werden als aktorische Stimuli heute ausschließlich elektrische Reizsignale verwendet. Nach der Implantation eines CI bei völlig ertaubten beziehungsweise gehörlosen Patienten ist im Regelfall ein Training zur Hörrehabilitation notwendig, da die artifiziellen Reize gelernt werden müssen, weil sie prinzipiell nicht der biologisch adäquaten Reizform des Innenohres entsprechen. Dem gegenüber entfällt diese Rehabilitationsphase nach Implantation eines elektromechanischen Hörsystems bei Schwerhörigen, da die mechanische Reizform wie oben beschrieben biologisch adäquat ist und letztendlich einer Versorgung mit einem Hörgerät zumindest bezüglich der grundsätzlichen Funktion weitgehend entspricht, das heißt, der stimulierende Reiz am Eingang des Innenohres ist vibratorischer Natur.

Bei an Taubheit grenzender Schwerhörigkeit sind aus den genannten Gründen unzulänglichen Lautstärkepegels implantierbare, elektromechanische Systeme bislang nicht einsetzbar; hier kommen Cochlea Implantate mit rein elektrischer Reizung des Innenohres in Frage, die naturgemäß keine Klangqualität erwarten lassen, die z.B. eine akzeptable Musikübertragung ermöglicht, sondern vorrangig zur Erlangung beziehungsweise Wiederherstellung eines ausreichenden Sprachverständnisses möglichst ohne Lippenablesen konzipiert sind. Aufgrund der elektrischen Reizung sind, wie beschrieben, Hörverluste in einem spektral breiten audiologischen Bereich möglich, die bis zur vollständigen Ertaubung reichen.

Seit kurzer Zeit ist aus CI-Implantationen wissenschaftlich bekannt, daß auch bei nicht vollständiger Taubheit Cochlea-Implantate (Cis) erfolgreich angewendet werden können, wenn mit einem konventionellen Hörgerät keine ausreichende Sprachdiskrimination mehr zu erreichen ist. Interessanterweise konnte nachgewiesen werden, daß die wesentlichen Innenohrstrukturen, die die akustische Resthörigkeit ermöglichen, zum Teil oder weitgehend langzeitstabil erhalten werden können, wenn eine CI-Elektrode in die Cochlea eingeführt wird (Ruh, S. et al.: "Cochlear Implant bei resthörigen Patienten", Laryngo-Rhino-Otol. 76 (1997), 347-350; Müller-Deile, J. et al.: "Cochlear-Implant-Versorgung bei nicht tauben Patienten?", Laryngo-Rhino-Otol. 77 (1998), 136-143; Lehnhardt, E.: "Intracochlear placement of cochlear implant electrodes in soft surgery technique", HNO 41 (1993), 356-359). In absehbarer Zeit werden CI-Elektroden bei Resthörigkeit klinisch sicher so intracochleär plaziert werden können, daß die verbleibenden Innenohrstrukturen langzeitstabil erhaltbar sind und somit auch auf biologisch adäquatem Weg, das heißt vibratorisch, weiterhin stimulierbar sind und zu einem verwertbaren Höreindruck führen.

Der Erfindung liegt die Aufgabe zugrunde, ein mindestens teilweise implantierbares System zur Rehabilitation einer Hörstörung zu schaffen, das besonders gut und flexibel an die individuelle pathologische und audiologische Situation des jeweiligen Patienten angepaßt werden kann.

Diese Aufgabe wird dadurch gelöst, daß bei einem mindestens teilweise implantierbaren System zur Rehabilitation einer Hörstörung, das mindestens einen Sensor (Mikrofon) zur Aufnahme des Schallsignals und dessen Umwandlung in entsprechende elektrische Signale, eine elektronische Signalverarbeitungseinheit zur Audiosignalverarbeitung und - verstärkung, eine elektrische Energieversorgungseinheit, welche einzelne Komponenten des Systems mit Strom versorgt, sowie eine ausgangsseitige aktorische Stimulationsanordnung umfasst, erfindungsgemäß als aktorische Stimulationsanordnung eine duale intracochleäre Anordnung vorgesehen ist, die in Kombination eine Stimulatoranordnung mit mindestens einem Stimulatorelement zur mindestens mittelbaren mechanischen Stimulation des Innenohres und eine elektrisch wirkende Reizelektrodenanordnung mit mindestens einer Cochlea-Implant-Elektrode zur elektrischen Stimulation des Innenohres aufweist.

Mit dem erfindungsgemäßen System können beide Stimulationsverfahren, das heißt, die Nutzung mechanischer und elektrischer Reize, in einem einzigen Implantatsystem zur Anwendung kommen und je nach individueller audiologischer Situation patientenspezifisch appliziert werden ("duales" Hörimplantat). Durch eine direkte Stimulation der Cochlea wird außerdem das Auftreten von Rückkopplungen, das heißt die Einkopplung des Ausgangssignals in den Sensor (Mikrofon), vermieden oder weitgehend reduziert, weil die Ossikelkette und damit das Trommelfell nicht beziehungsweise deutlich reduziert zu Schwingungen angeregt wird. Dies ist insbesondere dann von Vorteil, wenn ein Schallsensor (Mikrofonfunktion) in unmittelbarer Nähe zum Trommelfell appliziert wird, wie dies aus US-A-5 814 095 und US-A 5 999 632 bekannt ist. Insgesamt werden also mit dem vorliegenden Implantatsystem durch Anwendung eines kombinierten, "dualen" ausgangsseitigen Wandlerarrays die bekannten Nachteile eines rein elektromechanisch wirkenden, implantierbaren Hörsystems und eines rein elektrisch wirkenden, implantierbaren CIs so umgangen oder mindestens gemindert, daß durch eine kombinatorische Wirkung beider Reizformen und individuell maximierte Parametereinstellung des steuernden elektronischen Vorverarbeitungssystems einerseits eine audiologisch möglichst weite Indikationsstellung und andererseits im individuellen Patienten ein möglichst optimales Ergebnis bezüglich Sprachdiskrimination, ausreichender Lautstärke in allen relevanten spektralen Bereichen und hoher Klangqualität erreicht werden.

Vorteilhafte weitere Ausgestaltungen der Erfindung ergeben sich aus den Unteransprüchen.

Die Stimulatoranordnung kann vorteilhaft mindestens einen intracochleären elektromechanischen Wandler zur unmittelbaren mechanischen Stimulation des Innenohres und/oder mindestens eine intracochleäre Haarzellen-Reizelektrode zur mittelbaren mechanischen Stimulation des Innenohres durch elektrische Reizung von äußeren Haarzellen aufweisen. Vorzugsweise ist ein intracochleäres Array mit mehreren Stimulatorelementen zur unmittelbaren und/oder mittelbaren mechanischen Stimulation des Innenohres vorgesehen, das ebenso wie ein vorzugsweise mit einer Mehrzahl von Cochlea-Implant-Elektroden versehenes Cochlea-Implant-Elektrodenarray unmittelbar in einen flüssigkeitsgefüllten Raum des Innenohres (insbesondere scala tympani oder scala vestibuli) implantiert werden kann.

Die zur mittelbaren mechanischen Stimulation des Innenohres bestimmten Haarzellen-Reizelektroden sind vorzugsweise so ausgebildet, daß sie im implantierten Zustand in unmittelbarer Nähe der äußeren Haarzellen zu liegen kommen. Dabei läßt sich der Umstand nutzen, daß die äußeren Haarzellen mechanisch mit Längenänderungen reagieren, wenn sie artifiziell elektrisch gereizt werden. Diese dynamischen Elongationen sind bis zu sehr hohen Frequenzen, die bis über den zu versorgenden audiologischen Bereich reichen können, synchron mit dem elektrischen Reizsignal. Auf diese Weise lassen sich daher, ähnlich wie bei Verwendung von intracochleären elektromechanischen Wandlern, ausgangsseitig zum Beispiel mittels Cochlear-Implant-Elektroden erzielte elektrische Reizformen mit mechanischen cochleären Reizen kombinieren, weil die äußeren Haarzellen so als "elektromechanische Wandler" direkt betrieben werden, wie es bei einem gesunden, ungeschädigten Innenohres biologisch durch neuronale, efferente Ansteuerung geschieht. Diese Applikation eignet sich insbesondere für pathologische Fälle, bei denen nur die äußeren Haarzellen zum Beispiel durch ototoxische Medikamention geschädigt sind, das heißt die äußeren Haarzellen ihre neuronale Transmitterfunktion nicht mehr oder nur eingeschränkt ausführen können, die Stereozilien, die die Haarzellen "mechanisch" mit der Tektorialmembran verbinden, jedoch ungeschädigt sind oder noch so weit intakt sind, daß eine sichere, langzeistabile mechanische Kopplung zur Tektorialmembran gegeben ist.

Die zur unmittelbaren elektrischen Stimulation des Innenohres vorgesehene(n) Cochlea-Implant-Elektrode(n) ist (sind) zweckmäßig, wie aus dem Stand der Technik für Cochlea Implantate bekannt ist, so in einen Träger eingebettet oder an dem Träger fixiert, daß im implantierten Zustand ein Oberflächenanteil jeder Reizelektrode in direktem galvanischem Kontakt zu der lymphatischen Flüssigkeit des Innenohres oder direkt zu einer der zu reizenden neuronalen Strukturen steht.

Es kann ein gemeinsamer Träger für die Cochlea-Implant-Elektrode(n) und den oder die elektromechanische(n) Wandler vorgesehen sein, wobei elektromechanische Wandler und Cochlea-Implant-Elektroden vorteilhaft entlang dem Träger einander abwechselnd angeordnet sind. Dadurch kann die mechanische Wellenausbreitung innerhalb des Trägers zwischen benachbarten elektromechanischen Wandlerelementen bedämpft und eine örtlich konzentrierte mechanische Stimulation je Wandler erreicht werden.

Für die Cochlea-Implant-Elektrode(n) und den oder die elektromechanische(n) Wandler können aber auch separate Träger vorgesehen sein. In einem solchen Fall ist der Träger für die Cochlea-Implant-Elektrode(n) vorzugsweise so angeordnet, daß er im implantierten Zustand bezüglich der Cochlea nach innen orientiert ist, um möglichst nahe an den zu reizenden neuronalen Strukturen (Modiolus) zu liegen.

Die duale intracochleäre Anordnung hat vorzugsweise einen Gesamtdurchmesser im Bereich von 0,4 mm (apikaler Bereich) bis 2,0 mm (basaler Bereich) und eine Gesamtlänge zwischen 5 mm und 50 mm. Der oder die Träger bestehen vorteilhaft aus einem biokompatiblen und im Innenohr biostabilen Material, vorzugsweise einem Polymer, insbesondere einem Silikon. Die einzelnen elektromechanischen Wandler können dabei aus Biokompatibilitätsgründen so in den Träger eingebettet sein, daß sie von einer dünnen Schicht des Trägermaterials vollständig umgeben sind.

Um eine mechanische Wellenausbreitung von einem Wandler innerhalb des Trägers zu benachbarten Wandlern zu minimieren, sind in weiterer Ausgestaltung der Erfindung zwischen den einzelnen elektromechanischen Wandlern mechanische Dämpfungselemente in den Träger eingebettet, wobei das Material der Dämpfungselemente bei ähnlicher Querschnittsgeometrie wie die des Trägers vorzugsweise so gewählt ist, daß zur Erzielung hoher Dämpfungswerte ein großer mechanischer Impedanzunterschied zu dem Trägermaterial besteht. Wie bereits erwähnt, können gegebenenfalls die Cochlear-Implant-Elektroden zugleich als Dämpfungselemente genutzt werden.

Der beziehungsweise die elektromechanische(n) Wandler ist (sind) bevorzugt hermetisch dicht aufgebaut, und sie können grundsätzlich nach jedem bekannten elektromechanischen Wandlerprinzip arbeiten und insbesondere als elektromagnetische, elektrodynamische, piezoelektrische, magnetostriktive oder dielektrische (kapazitive) Wandler ausgebildet sein. Bei Anwendung des piezoelektrischen Wandlerprinzips ist (sind) der (die) elektromechanische(n) Wandler vorteilhaft unter Verwendung von PZT-Keramik (Blei-Zirkonat-Titanat) oder von PVDF (Polyvinylidenfluorid) aufgebaut, und zwar vorzugsweise mit Ausnützung von geometrischen Gestalttransformationen, insbesondere des Bimorph-Prinzips, des Unimorph-Prinzips oder des Heteromorph-Prinzips mit passiven Materialpartnern, so, daß bei gegebener Wandlerspannung eine maximale Auslenkung bei minimaler elektrischer Leistungsaufnahme erzeugt wird.

In weiterer Ausgestaltung der Erfindung sind mehrere Stimulatorelemente zur mindestens mittelbaren mechanischen Stimulation des Innenohres äquidistant oder in logarithmischen Distanzen - entsprechend der tonotopen Frequenz-Orts-Zuordnung längs der Basilarmembran des Innenohres - verteilt angeordnet, wobei im Falle einer tonotopen Anordnung der Stimulatorelemente zur mechanischen Stimulation des Innenohres 20 bis 24 Stimulatorelemente oder Gruppen von Stimulatorelementen gemäß den psychoakustischen Frequenzgruppen zu besonders günstigen Ergebnissen führen können.

Der beziehungsweise die elektromechanische(n) Wandler hat (haben) zweckmäßig einen Übertragungsbereich von etwa 100 Hz bis etwa 10 kHz, und er (sie) ist (sind) vorzugsweise hoch abgestimmt, das heißt, die erste mechanische Resonsanzfrequenz liegt am oberen Ende des angestrebten Übertragungsfrequenzbereiches, insbesondere bei etwa 8 kHz bis etwa 10 kHz. Dadurch wird erreicht, daß der Auslenkungsfrequenzgang der Wandler im Übertragungsbereich weitgehend frei ist von Resonanzen und bei Spannungseinprägung und Verwendung piezoelektrischer Wandler frequenzunabhängig eben. Im Übertragungsbereich tritt somit keine Welligkeit auf.

Die die Cochlea-Implant-Elektrode(n) und/oder die Haarzellen-Reizelektrode(n) können aus allen bekannten biokompatiblen Metallen bestehen, insbesondere aus reinem Platin, vorzugsweise aus Platin-Iridium-Legierungen (vorzugsweise 90 % Pt, 10 % Ir), reinem Gold, Goldlegierungen, Tantal, Tantallegierungen, Niob, Nioblegierungen sowie Edelstählen.

Die Signalverarbeitungseinheit weist zweckmäßig eine Vorverarbeitungsanordnung zum Vorverstärken und/oder Filtern sowie zum Analog-Digital- (A/D-) Wandeln der Schallsensorsignale auf. Sie kann insbesondere einen Antialiasing-Filter umfassen. Bei Vorhandensein mehrerer Schallsensoren ist vorzugsweise jedem der Schallsensoren ein eigener Analog-Digital-Wandler nachgeschaltet.

In weiterer Ausgestaltung der Erfindung kann die Signalverarbeitungseinheit Softwaremodule enthalten, die parallel zum Hörgerätebetrieb die Maskierung eines Tinnitus ermöglichen. Mit dem vorliegenden mehrkanaligen Hörimplantatsystem kann ein zumindest peripher zu lokalisierender Tinnitus effektiver maskiert werden als mit bekannten, konventionellen Tinnitus-Maskern.

Die Signalverarbeitungseinheit weist vorteilhaft einen digitalen Signalprozessor zum Verarbeiten der A/D-gewandelten und gegebenenfalls mittels der Vorverarbeitungsanordnung vorverarbeiteten Schallsensorsignale und/oder zum Generieren von digitalen Signalen für eine Tinnitusmaskierung auf, wobei der aktorischen Stimulationsanordnung mindestens ein Digital-Analog-Wandler zugeordnet ist und vorzugsweise dem beziehungsweise den Stimulatorelement(en) zur mindestens mittelbaren mechanischen Stimulation des Innenohres und der beziehungsweise den Cochlea-Implant-Elektrode(n) zur elektrischen Stimulation des Innenohres jeweils ein eigener Digital-Analog-Wandler vorgeschaltet ist.

In weiterer Ausgestaltung der Erfindung enthält die Signalverarbeitungselektronik Softwaremodule, welche die Ansteuerung der Stimulatorelemente zur mindestens mittelbaren mechanischen Stimulation des Innenohres, also insbesondere der das Innenohr unmittelbar mechanisch anregenden intracochleären elektromechanischen Wandler und/oder der das Innenohr im Zusammenwirken mit den äußeren Haarzellen mechanisch anregenden Haarzellen-Reizelektroden, so vornehmen, daß die spektralen, zeitlichen, amplituden- und phasenbezogenen Eigenschaften der die Stimulatorelemente ansteuernden Signale so bemessen sind, daß auf der Basilarmembran des geschädigten Innenohres eine Wanderwelle erzeugt wird, die der eines gesunden Gehörs möglichst nahe kommt.

Die Softwaremodule können dabei statisch in der Weise ausgelegt sein, daß sie aufgrund wissenschaftlicher Erkenntnisse einmalig in einem Programmspeicher des digitalen Signalprozessors abgelegt werden und unverändert bleiben. Liegen dann aber später zum Beispiel aufgrund neuerer wissenschaftlicher Erkenntnisse verbesserte Algorithmen zur Sprachsignalaufbereitung und -verarbeitung vor und sollen diese genutzt werden, muß durch einen invasiven, operativen Patienteneingriff das gesamte Implantat oder das Implantatmodul, das die entsprechende Signalverarbeitungseinheit enthält, gegen ein neues mit der veränderten Betriebssoftware ausgetauscht werden. Dieser Eingriff birgt erneute medizinische Risiken für den Patienten und ist mit hohem Aufwand verbunden.

Diesem Problem kann dadurch begegnet werden, daß in weiterer Ausgestaltung der Erfindung eine drahtlose, bevorzugt PC-basierte, Telemetrieeinrichtung zur Übertragung von Daten zwischen dem implantierten Teil des Systems und einer externen Einheit, insbesondere einem externen Programmiersystem vorgesehen ist, dem Signalprozessor zur Aufnahme und Wiedergabe eines Betriebsprogramms eine wiederholt beschreibbare, implantierbare Speicheranordnung zugeordnet ist und mindestens Teile des Betriebsprogramms durch von der externen Einheit über die Telemetrieeinrichtung übermittelte Daten geändert oder ausgetauscht werden können. Auf diese Weise läßt sich nach Implantation des implantierbaren Systems die Betriebssoftware als solche verändern oder auch vollständig austauschen, wie dies für im übrigen bekannte Systeme zur Rehabilitation von Hörstörungen in der DE-C-199 15 846.0 erläutert ist.

Bevorzugt ist die Auslegung so beschaffen, daß darüberhinaus bei vollimplantierbaren Systemen auch in an sich bekannter Weise Betriebsparameter, das heißt patientenspezifische Daten, wie beispielsweise audiologische Anpaßdaten, oder veränderbare Implantatsystemparameter (zum Beispiel als Variable in einem Softwareprogramm zur Regelung einer Batterienachladung) nach der Implantation transkutan, das heißt drahtlos durch die geschlossene Haut, in das Implantat übertragen und damit verändert werden können. Dabei sind die Softwaremodule bevorzugt dynamisch, oder mit anderen Worten lernfähig, ausgelegt, um der Ausbildung einer Wanderwellenkonfiguration, welche die Art der Wanderwellenausbildung eines gesunden, nicht geschädigten Innenohres approximiert, möglichst optimal nahe zu kommen. Insbesondere können die Softwaremodule adaptiv ausgelegt sein, und eine Parameteranpassung kann durch "Training" durch den Implantatträger und weitere Hilfsmittel vorgenommen werden.

Weiterhin kann die Signalverarbeitungselektronik ein Softwaremodul enthalten, das eine möglichst optimale Simulation eines "gesunden" cochleären Verstärkers auf der Basis eines lernfähigen neuronalen Netzwerkes erreicht. Das Training dieses neuronalen Netzwerks kann wieder durch den Implantatträger erfolgen und/oder unter Zuhilfenahme weiterer externer Hilfsmittel. Insbesondere kann in dem neuronalen Netzwerk zur Simulation eines "gesunden" cochleären Verstärkers das Prinzip der "Time-Reversed Acoustics" (TRA) implementiert sein, und die Ansteuerung der Stimulatorelemente zur mechanischen Stimulation des Innenohres kann mittels TRA so erfolgen, daß örtlich begrenzte Bereiche der Cochlea mechanisch stimuliert werden.

Die Speicheranordnung zum Speichern von Betriebsparametern und die Speicheranordnung zur Aufnahme und Wiedergabe des Betriebsprogramms können als voneinander unabhängige Speicher implementiert sein; es kann sich jedoch auch um einen einzigen Speicher handeln, in dem sowohl Betriebsparameter als auch Betriebsprogramme abgelegt werden können.

Die vorliegende Lösung erlaubt eine Anpassung des Systems an Gegebenheiten, die erst nach Implantation des implantierbaren Systems zu erfassen sind. So sind beispielsweise bei einem mindestens teilweise implantierbaren Hörsystem zur Rehabilitation einer monauralen oder binauralen Innenohrstörung sowie eines Tinnitus mit mechanischer Stimulation des Innenohres die sensorischen (Schallsensor beziehungsweise Mikrofon) und aktorischen (Ausgangsstimulator) biologischen Schnittstellen immer abhängig von den anatomischen, biologischen und neurophysiologischen Gegebenheiten, z.B. von dem interindividuellen Einheilprozeß. Diese Schnittstellenparameter können individuell insbesondere auch zeitvariant sein. So können beispielsweise das Übertragungsverhalten eines implantierten Mikrofons aufgrund von Gewebebelägen und das Übertragungsverhalten eines an das Innenohr angekoppelten elektromechanischen Wandlers aufgrund unterschiedlicher Ankopplungsqualität interindividuell und individuell variieren. Solche Unterschiede der Schnittstellenparameter, die sich bei den aus dem Stand der Technik bekannten Vorrichtungen nicht einmal durch den Austausch des Implantats mindern beziehungsweise eliminieren ließen, können vorliegend durch Veränderung beziehungsweise Verbesserung der Signalverarbeitung des Implantats optimiert werden.

Bei einem mindestens teilweise implantierbaren Hörsystem kann es sinnvoll oder notwendig werden, nach Implantation verbesserte Signalverarbeitungsalgorithmen zu implementieren. Dabei sind insbesondere zu nennen:
- Sprachanalyseverfahren (z.B. Optimierung einer Fast-Fourier-Transformation (FFT)),
- statische oder adaptive Störschallerkennungsverfahren,
- statische oder adaptive Störschallunterdrückungsverfahren,
- Verfahren zur Optimierung des systeminternen Signal-Rauschabstandes,
- optimierte Signalverarbeitungsstrategien bei progredienter Hörstörung,
- ausgangspegelbegrenzende Verfahren zum Schutz des Patienten bei Implantatfehlfunktionen beziehungsweise externen Fehlprogrammierungen,
- Verfahren zur Vorverarbeitung mehrerer Sensor(Mikrofon)signale, insbesondere bei binauraler Positionierung der Sensoren,
- Verfahren zur binauralen Verarbeitung zweier oder mehrerer Sensorsignale bei binauraler Sensorpositionierung z.B. Optimierung des räumlichen Hörens beziehungsweise Raumorientierung,
- Phasen- beziehungsweise Gruppenlaufzeit-Optimierung bei binauraler Signalverarbeitung,
- Verfahren zur optimierten Ansteuerung der Ausgangsstimulatoren, insbesondere bei binauraler Positionierung der Stimulatoren

Mit dem vorliegenden System lassen sich auch nach der Implantation unter anderem die folgenden Signalverarbeitungsalgorithmen implementieren:
- Verfahren zur Rückkopplungsunterdrückung beziehungsweise -minderung,
- Verfahren zur Optimierung des Betriebsverhaltens des beziehungsweise der Ausgangswandler (z.B. Frequenz- und Phasengangoptimierung, Verbesserung des Impulsübertragungsverhaltens),
- Sprachsignal-Kompressionsverfahren bei Innenohrschwerhörigkeiten,
- Signalverarbeitungsmethoden zur Recruitment-Kompensation bei Innenohrschwerhörigkeiten.

Des weiteren ist bei Implantatsystemen mit einer sekundären Energieversorgungseinheit, das heißt einem nachladbaren Akkumulatorsystem, aber auch bei Systemen mit primärer Batterieversorgung davon auszugehen, daß diese elektrischen Energiespeicher mit voranschreitender Technologie immer größere Lebensdauern und damit steigende Verweilzeiten im Patienten ermöglichen. Es ist davon auszugehen, daß die Grundlagen- und Applikationsforschung für Signalverarbeitungsalgorithmen schnelle Fortschritte macht. Die Notwendigkeit oder der Patientenwunsch einer Betriebssoftwareanpassung beziehungsweise -veränderung wird daher voraussichtlich vor Ablauf der Lebensdauer der implantatinternen Energiequelle eintreten. Das vorliegend beschriebene System erlaubt eine derartige Anpassung der Betriebsprogramme des Implantats auch im bereits implantierten Zustand.

Vorzugsweise ist ferner eine Zwischenspeicheranordnung vorgesehen, in welcher von der externen Einheit über die Telemetrieeinrichtung übermittelte Daten vor dem Weiterleiten an den Signalprozessor zwischengespeichert werden können. Auf diese Weise läßt sich der Übertragungsvorgang von der externen Einheit zu dem implantierten System abschließen, bevor die über die Telemetrieeinrichtung übermittelten Daten an den Signalprozessor weitergeleitet werden.

Des weiteren kann eine Überprüfungslogik vorgesehen sein, die in der Zwischenspeicheranordnung gespeicherte Daten vor dem Weiterleiten an den Signalprozessor einer Überprüfung unterzieht. Es kann ein Mikroprozessorbaustein, insbesondere ein Microcontroller, zum implantatinternen Steuern der A/D-Wandler und/oder der D/A-Wandler und/oder des Signalprozessors über einen Datenbus vorgesehen sein, wobei zweckmäßig die Überprüfungslogik und die Zwischenspeicheranordnung in dem Mikroprozessorbaustein implementiert sind und wobei über den Datenbus und die Telemetrieeinrichtung auch Programmteile oder ganze Softwaremodule zwischen der Außenwelt, dem Mikroprozessorbaustein und dem Signalprozessor übermittelt werden können.

Dem Mikroprozessorbaustein ist vorzugsweise eine implantierbare Speicheranordnung zum Speichern eines Arbeitsprogramms für den Mikroprozessorbaustein zugeordnet, und mindestens Teile des Arbeitsprogramms für den Mikroprozessorbaustein können durch von der externen Einheit über die Telemetrieeinrichtung übermittelte Daten geändert oder ausgetauscht werden.

In weiterer Ausgestaltung der Erfindung können mindestens zwei Speicherbereiche zur Aufnahme und Wiedergabe mindestens des Betriebsprogramms des Signalprozessors vorgesehen sein. Dies trägt zur Fehlersicherheit des Systems bei, indem durch das mehrfache Vorhandensein des Speicherbereichs, welcher das beziehungsweise die Betriebsprogramme enthält, beispielsweise nach einer Übertragung von extern oder aber beim Einschalten des Implantates eine Überprüfung der Fehlerfreiheit der Software durchgeführt werden kann.

Analog hierzu kann auch die Zwischenspeicheranordnung mindestens zwei Speicherbereiche zur Aufnahme und Wiedergabe von von der externen Einheit über die Telemetrieeinrichtung übermittelten Daten aufweisen, so daß nach einer Datenübertragung von der externen Einheit noch im Bereich des Zwischenspeichers eine Überprüfung der Fehlerfreiheit der übermittelten Daten vorgenommen werden kann. Die Speicherbereiche können zur beispielsweise komplementären Ablage der von der externen Einheit übermittelten Daten ausgelegt sein. Mindestens einer der Speicherbereiche der Zwischenspeicheranordnung kann aber auch zur Aufnahme nur eines Teils der von der externen Einheit übermittelten Daten ausgelegt sein, wobei in diesem Fall die Überprüfung der Fehlerfreiheit der übermittelten Daten abschnittsweise erfolgt.

Um zu gewährleisten, daß bei Übertragungsfehlern ein erneuter Übertragungsvorgang gestartet werden kann, kann dem Signalprozessor ferner ein vorprogrammierter, nicht überschreibbarer Festspeicherbereich zugeordnet sein, in welchem die für einen "Minimalbetrieb" des Systems erforderlichen Anweisungen und Parameter gespeichert sind, beispielsweise Anweisungen, die nach einem "Systemabsturz" zumindest einen fehlerfreien Betrieb der Telemetrieeinrichtung zum Empfang eines Betriebsprogramms sowie Anweisungen zum Einspeichern desselben in die Steuerlogik gewährleisten.

Wie bereits erwähnt, ist die Telemetrieeinrichtung in vorteilhafter Weise außer zum Empfang von Betriebsprogrammen von der externen Einheit auch zur Übermittlung von Betriebsparametern zwischen dem implantierbaren Teil des Systems und der externen Einheit ausgelegt, so daß einerseits solche Parameter von einem Arzt, einem Hörgeräteakustiker oder dem Träger des Systems selbst eingestellt werden können (z.B. Lautstärke), andererseits das System aber auch Parameter an die externe Einheit übermitteln kann, beispielsweise um den Status des Systems zu überprüfen.

Ein vollständig implantierbares Hörsystem der vorliegend erläuterten Art kann implantatseitig neben der aktorischen Stimulationsanordnung und der Signalverarbeitungseinheit mindestens einen implantierbaren Schallsensor und ein nachladbares elektrisches Speicherelement aufweisen, wobei in einem solchen Fall eine drahtlose, transkutane Ladevorrichtung zum Laden des Speicherelements vorgesehen sein kann. Es versteht sich jedoch, daß zur Energieversorgung auch eine Primärzelle oder eine andere Energieversorgungseinheit vorhanden sein kann, die keine transkutane Nachladung benötigt. Dies gilt insbesondere, wenn man berücksichtigt, daß in naher Zukunft vor allem durch Weiterentwicklung der Prozessortechnologie mit wesentlicher Verminderung des Energiebedarfs für elektronische Signalverarbeitung zu rechnen ist, so daß für implantierbare Hörsysteme neue Energieversorgungsformen praktisch anwendbar werden, zum Beispiel eine den Seebeck-Effekt nutzende Energieversorgung, wie sie in DE-C-198 27 898 beschrieben ist. Vorzugsweise ist auch eine drahtlose Fernbedienung zur Steuerung der Implantatfunktionen durch den Implantatträger vorhanden.

Bei teilimplantierbarer Ausbildung des Hörsystems sind zweckmäßig mindestens ein Schallsensor, die elektronische Anordnung zur Audiosignalverarbeitung und - verstärkung, die Energieversorgungseinheit sowie eine Modulator/Sender-Einheit in einem extern am Körper, vorzugsweise am Kopf über dem Implantat, zu tragenden externen Modul enthalten. Das Implantat weist die intracochleäre aktorische Stimulatoranordnung auf, ist aber energetisch passiv und empfängt seine Betriebsenergie sowie Steuerdaten für die intracochleäre aktorische Stimulatoranordnung über die Modulator/Sender-Einheit im externen Modul.

Das beschriebene System kann monaural oder binaural ausgelegt sein. Ein binaurales System zur Rehabilitation einer Hörstörung beider Ohren weist zwei Systemeinheiten auf, die jeweils einem der beiden Ohren zugeordnet sind. Dabei können die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, daß in beiden Systemeinheiten eine optimierte binaurale Signalverarbeitung und Wandler-Array-Ansteuerung erreicht wird.

Bevorzugte Ausführungsformen des erfindungsgemäßen Systems sind nachstehend unter Bezugnahme auf die beiliegenden Zeichnungen näher beschrieben. Es zeigen:
- Fig. 1: schematisch ein Ausführungsbeispiel eines intracochleären, dualen Stimulationsarrays,
- Fig. 2: ein Detail der Fig. 1,
- Fig. 3: schematisch ein weiteres Ausführungsbeispiel eines intracochleären, dualen Stimulationsarrays,
- Fig. 4: schematisch und in gegenüber den Figuren 1 und 3 vergrößerter Darstellung eine Anordnung, bei der zur mechanischen Stimulation des Innenohres intracochleäre miniaturisierte elektrische Reizelektroden vorgesehen sind, die mit äußeren Haarzellen zusammenwirken,
- Fig. 5: schematisch ein Ausführungsbeispiel für den Aufbau eines signalverarbeitenden Elektronikmoduls eines mindestens teilweise implantierbaren Hörsystems,
- Fig. 6: schematisch ein Ausführungsbeispiel für den Aufbau eines vollständig implantierbaren Hörsystems mit einem intracochleären, dualen Stimulationsarray gemäß Fig. 1,
- Fig. 7: schematisch ein Ausführungsbeispiel für den Aufbau eines teilimplantierbaren Hörsystems mit einem intracochleären, dualen Stimulationsarray gemäß Fig. 1,
- Fig. 8: eine binaurale Anwendung eines Hörimplantates gemäß Fig. 6, bei dem die Signalverarbeitungsmodule über eine drahtgebundene implantierbare Leitungsverbindung miteinander kommunizieren,
- Fig. 9: eine binaurale Anwendung eines Hörimplantates gemäß Fig. 6, bei dem die Signalverarbeitungsmodule über eine drahtlose Verbindung miteinander kommunizieren,
- Fig. 10: eine binaurale Anwendung eines Hörimplantates gemäß Fig. 6, bei dem die Signalverarbeitungsmodule über eine körperschallgekoppelte Ultraschallstrecke miteinander kommunizieren, sowie
- Fig. 11: eine binaurale Anwendung eines Hörimplantates gemäß Fig. 6, bei dem die Signalverarbeitungsmodule über eine das Gewebe des Implantatträgers einschließende Übertragungsstrecke miteinander kommunizieren.

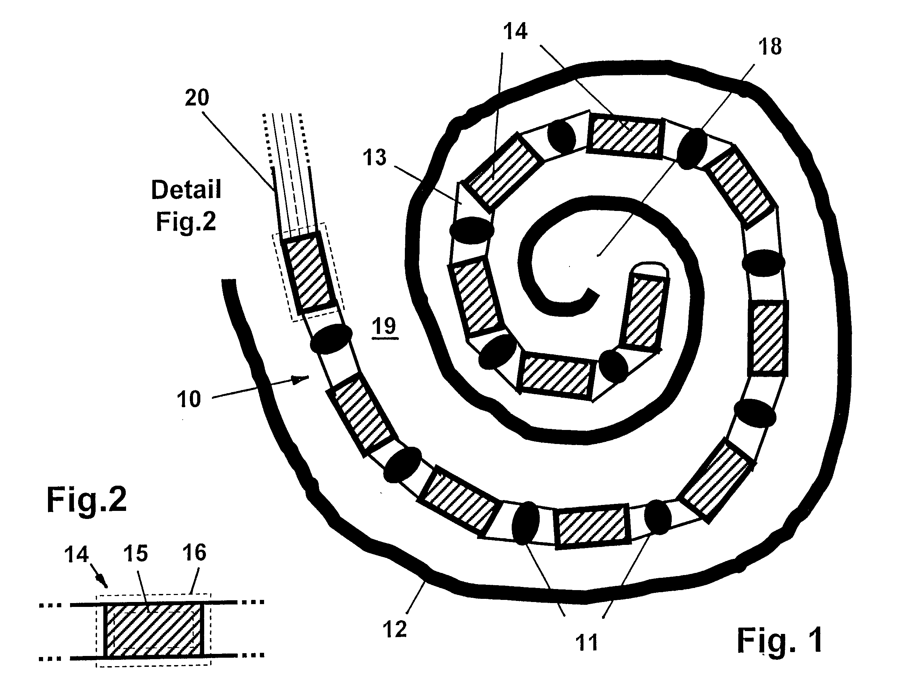

Fig. 1 zeigt schematisch ein intracochleäres, duales Stimulationsarray 10 mit mehreren Cochlear-Implant-Elektroden 11 zur unmittelbaren elektrischen Stimulation des Innenohres und mehreren intracochleären elektromechanischen Wandlern 14 zur unmittelbaren mechanischen Stimulation des Innenohres in einem gemeinsamen mechanischen Träger 13. Prinzipiell ist dieses Array ähnlich wie mehrkanaliges, intracochleäres Cochlea-Implantat-Elektrodenarray aufgebaut. Der Träger 13 besteht bevorzugt im wesentlichen aus einem flexiblen Silikonformteil von vorzugsweise kreisförmigem Querschnitt. Das Formteil wird durch das ovale Fenster, das runde Fenster oder eine artifizielle Eröffnung der Cochlea 12 oder in die flüssigkeitsgefüllten Innenohrräume geschoben. Die elektromechanischen Wandler 14 sind in Fig. 1 schematisch als zylinderförmige Elemente mit ebenfalls kreisförmigem Querschnitt gezeigt. Innerhalb des Trägers 13 befinden sich elektrische Zuleitungen zu den Cochlear-Implant-Elektroden 11 und den Wandlern 14; diese Zuleitungen sind nicht näher dargestellt.

Die elektromechanischen Wandler 14 arbeiten vorzugsweise nach dem Prinzip der dynamischen Volumenänderung aufgrund einer dynamischen Oberflächenvergrößerung beziehungsweise -verkleinerung entsprechend einem ansteuernden elektrischen Wandlerwechselspannungssignal. Eine solche dynamische Volumen- beziehungsweise Oberflächenveränderung ist schematisch in Fig. 2 dargestellt. Dort sind mit gestrichelten Linien 15 und 16 ein minimales beziehungsweise ein maximales Volumen angedeutet. Die erforderlichen Volumenveränderungen für einen adäquaten, äquivalenten Schalldruckpegel von etwa 100 dB SPL ergeben sich zu etwa 2 · 10⁻⁴ Mikroliter (US-A-5 772 575).

Die Wandler 14 sind beispielsweise äquidistant längs des Trägers 13 verteilt oder in logarithmischen Distanzen entsprechend der tonotopen Orts-Frequenz-Zuordnung längs der Basilarmembran des Innenohres. Der Gesamtdurchmesser der Wandler-Elektroden-Anordnung 10 liegt vorzugsweise im Bereich von 0,4 mm (apikaler Bereich 18 gemäß Fig. 1) bis 2,0 mm (basaler Bereich 19 gemäß Fig. 1). Die Gesamtlänge des Stimulationsarrays 10 liegt zweckmäßig zwischen 5 mm und 50 mm. Die elektromechanischen Wandlerelemente 14 sind aus Biokompatibilitätsgründen vorzugsweise so in den Träger 13 eingebettet, daß sie vollständig von einer dünnen Schicht des Trägermaterials umgeben sind (in Fig. 1 nicht dargestellt). Der Träger 13 des Stimulationsarrays 10 besteht aus einem biokompatiblen und im Innenohr biostabilen Material, vorzugsweise Polymere wie entsprechende Silikone.

Die Cochlear-Implant-Elektroden 11 sind, wie bei Cochlea-Implantaten bekannt, so in den Träger 13 eingebettet beziehungsweise in oder an dem Träger 13 fixiert, daß ein Oberflächenanteil je Reizelektrode in direktem galvanischem Kontakt zu der lymphatischen Flüssigkeit des Innenohres oder direkt zu einer zu reizenden neuronalen Struktur besteht. Die Cochlear-Implant-Elektroden 11 können aus allen bekannten biokompatiblen Metallen bestehen, insbesondere aus reinem Platin, vorzugsweise aus Platin-Iridium-Legierungen (vorzugsweise 90 % Pt, 10 % Ir), reinem Gold, Goldlegierungen, Tantal, Tantallegierungen, Niob, Nioblegierungen sowie Edelstählen.

Vorteilhaft sind die elektromechanischen Wandler 14 und die Cochlear-Implant-Elektroden 11 in Längsrichtung des Trägers 13 einander abwechselnd angeordnet, damit die mechanische Wellenausbreitung innerhalb des Trägers zwischen benachbarten elektromechanischen Wandlerelementen bedämpft wird und somit eine örtlich konzentrierte mechanische Stimulation je Wandler erreicht wird. Eine Wandlerarray-Zuleitung ist bei 20 angedeutet.

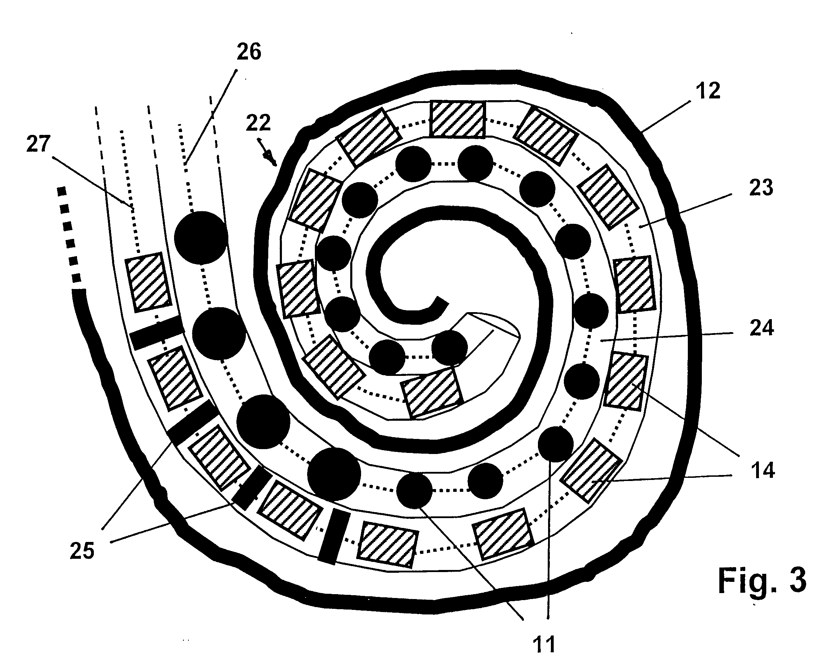

Fig. 3 zeigt schematisch ein weiteres Ausführungsbeispiel ein intracochleären, dualen Stimulationsarrays 22 mit mehreren elektromechanischen Wandlern 14 und Cochlear-Implant-Elektroden 11, bei dem abweichend von Fig. 1 die elektromechanischen Wandler 14 und die Cochlear-Implant-Elektroden 11 in zwei separaten Trägern 23 beziehungsweise 24 angeordnet sind. Die Träger 23, 24 können, wie dargestellt, mechanisch miteinander verbunden sein und werden so gemeinsam in die Cochlea 12 geführt, wobei vorzugsweise das Array mit den Cochlear-Implant-Elektroden 11 nach innen orientiert ist, um möglichst nahe an den zu reizenden neuronalen Strukturen (Modiolus) zu liegen. Die beiden Träger können aber auch mechanisch völlig unabhängig voneinander sein (nicht dargestellt); sie werden dann einzeln simultan oder nacheinander in die Cochlea 12 eingebracht. Elektrische Zuleitungen für die Cochlear-Implant-Elektroden 11 und die elektromechanischen Wandler 14 sind bei 26 beziehungsweise 27 angedeutet.

Zwischen den einzelnen Wandlern 14 des elektromechanischen Arrays können mechanische Dämpfungselemente 25 in den Träger eingebettet sein, die die mechanische Wellenausbreitung innerhalb des Trägers 23 zu den benachbarten Wandlerelementen 14 minimieren, ähnlich wie dies in Fig. 1 durch die zwischenangeordneten Cochlear-Implant-Elektroden 11 geschieht. Das Material dieser Dämpfungselemente 25 ist bei ähnlicher Querschnittsgeometrie wie der des Trägers 23vorzugsweise so gewählt, daß ein großer mechanischer Impedanzunterschied zu dem Trägermaterial besteht, um hohe Dämpfungswerte zu erhalten.

Die elektromechanischen Wandler 14 können nach jedem bekannten elektromechanischen Wandlerprinzip arbeiten, nämlich elektromagnetisch, elektrodynamisch, piezoelektrisch, magnetostriktiv oder dielektrisch (kapazitiv). Die elektromechanischen Wandler 14 können mit mikrosystemtechnischen Methoden entwickelt beziehungsweise hergestellt werden, die eine hohe Miniaturisierung und eine exzellente Reproduzierbarkeit der einzelnen Wandler gestatten. Diese Eigenschaften der mikrosystemtechnischen Erzeugung können besonders vorteilhaft sein, weil es erwartungsgemäß bei der beabsichtigten Funktion des Wandlerarrays auf einen Phasengleichlauf der einzelnen elektromechanischen Wandler ankommt. Ein möglicher, mikrosystemtechnischer Aufbau eines einzelnen Wandlers ist in der Patentliteratur von Ron Maynard angegeben (WO-A-99/03146).

Die einzelnen Wandler 14 werden von einem nachstehend näher erläuterten elektronischen Vorverarbeitungssystem vorzugsweise so angesteuert, daß durch jeweilige Wahl des spektralen Übertragungsbereiches je Wandler, der vibratorischen Amplitude und der Phasenlage der Wandler zueinander im aktorischen Gesamtergebnis der Innenohrstimulation eine Wanderwellenausbildung auf der Basilarmembran entsteht, die bei dem jeweiligen externen Schallereignis der Wanderwellenform möglichst nahekommt, die sich bei nicht geschädigtem cochleärem Verstärker und damit intakten äußeren Haarzellen ergeben würde.

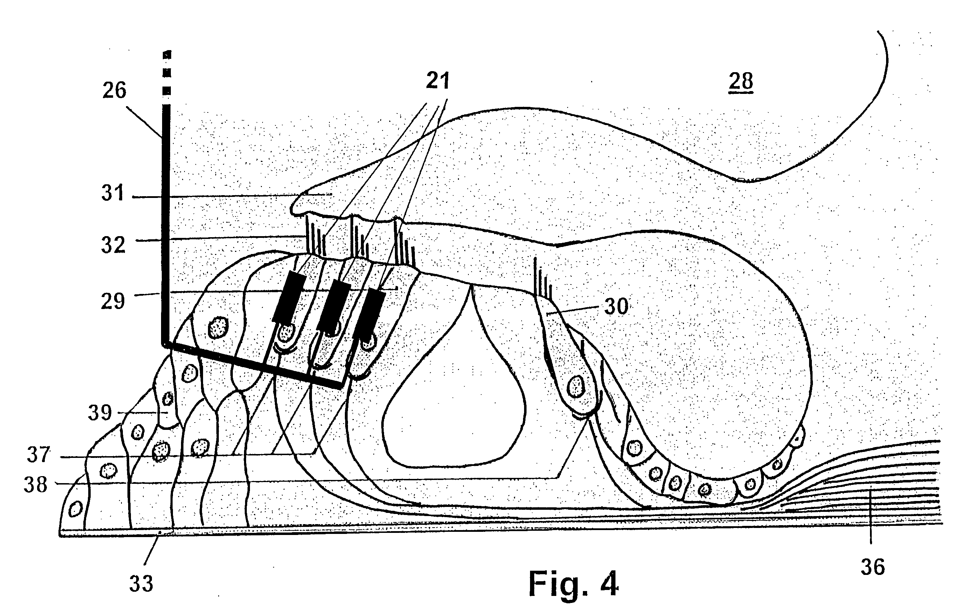

Fig. 4 zeigt in größerem Maßstab einen Teil des Innenohres. Dabei sind bei 28 ein mit intracochleärer Flüssigkeit angefüllter Innenohrraum, bei 29 und 30 äußere beziehungsweise innere Haarzellen, bei 31 die Tektorialmembran, bei 32 Stereozilien, bei 33 die Basilarmembran, bei 36 der Hörnerv, bei 37 und 38 efferente beziehungsweise afferente Synapsen und bei 39 Stützzellen angedeutet. Anstelle der elektromechanischen Wandler 14 zur unmittelbaren mechanischen Anregung des Innenohres gemäß den Figuren 1 bis 3 sind bei diesem Ausführungsbeispiel für eine mittelbare mechanische Stimulation des Innenohres intracochleäre Haarzellen-Reizelektroden 21 vorgesehen, die im implantierten Zustand in unmittelbarer Nähe der äußeren Haarzellen 29 liegen. Mittels der Haarzellen-Reizelektroden 21 erfolgt eine artifizielle elektrische Reizung der äußeren Haarzellen 29, und die Haarzellen 29 reagieren darauf mechanisch mit das Innenohr mechanisch stimulierenden Längenänderungen. Bei entsprechender Ansteuerung durch ein Prozessorsystem der unten erläuterten Art kann infolgedessen wiederum für die Ausbildung von Wanderwellen auf der Basilarmembran 33 gesorgt werden, die bei dem jeweiligen externen Schallereignis der Wanderwellenform möglichst nahekommen, die sich bei nicht geschädigtem cochleärem Verstärker ergeben würde.

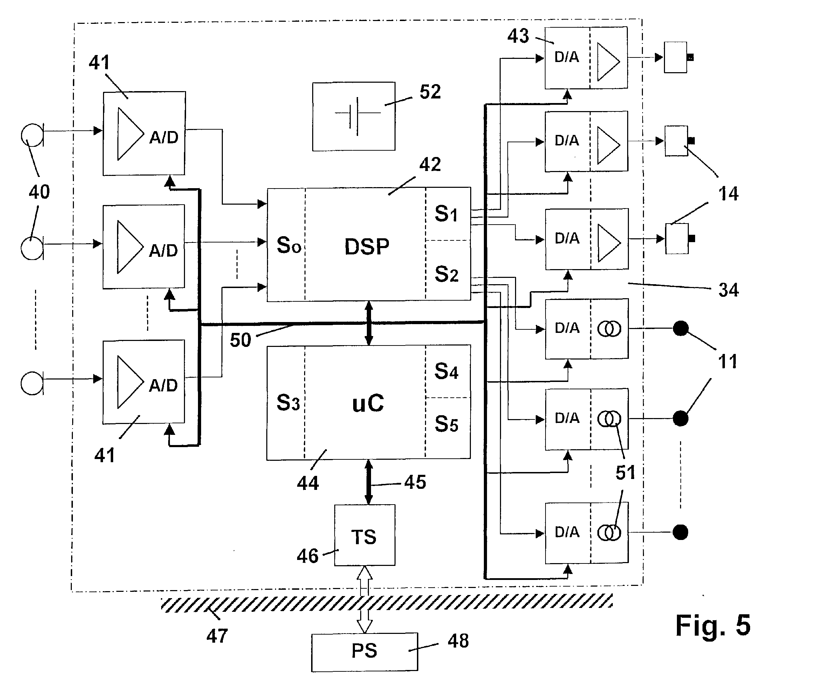

Fig. 5 zeigt den möglichen Aufbau des signalverarbeitenden Elektronikmoduls 34 des mindestens teilweise implantierbaren Hörsystems. Über einen oder mehrere Schallsensoren (Mikrofone) 40 wird das externe Schallsignal aufgenommen und in elektrische Signale umgewandelt. Die analogen elektrischen Sensorsignale werden an Module 41 geleitet, wo sie vorverarbeitet, insbesondere vorverstärkt, und in digitale Signale umgewandelt (A/D) werden. Diese Vorverarbeitung kann beispielsweise in einer analogen linearen oder nicht-linearen Vorverstärkung und Filterung (zum Beispiel Antialiasing-Filterung) bestehen.

Die digitalisierten Sensorsignale werden in einem digitalen Signalprozessor 42 (DSP) weiterverarbeitet. Der Signalprozessor 42 enthält einen nicht überschreibbaren Festspeicherbereich S₀, in welchem die für einen "Minimalbetrieb" des Systems erforderlichen Anweisungen und Parameter gespeichert sind, sowie Speicherbereiche S₁ und S₂, in denen die Betriebssoftware der bestimmungsgemäßen Funktion beziehungsweise Funktionen des Implantatsystems abgelegt sind. Die wiederholt beschreibbaren Programmspeicher S₁ und S₂ zur Aufnahme der Betriebssoftware können auf EEPROM-Basis oder statischen RAM-Zellen basieren, wobei im letztgenannten Fall dafür gesorgt sollte, daß dieser RAM-Bereich immer durch das implantatinterne Energieversorgungssystem "gepuffert" ist.

Die digitalen Ausgangssignale des Signalprozessors 42 werden in Digital-Analog-Wandlern (D/A) 43 in Analogsignale umgewandelt und verstärkt sowie dann den Cochlear-Implant-Elektroden 11 und den elektromechanischen Wandlern 14 beziehungsweise den Haarzellen-Reizelektroden 21 zugeführt. Die D/A-Wandler 43 können gegebenenfalls mindestens teilweise entfallen, wenn beispielsweise ein pulsweitenmoduliertes, serielles digitales Ausgangssignal des Signalprozessors 42 direkt an die Ausgangswandler 14 übermittelt wird und/oder für die Cochlear-Implant-Elektroden 11 beziehungsweise die Haarzellen-Reizelektroden 21 digital gesteuerte Stromquellen vorgesehen sind.

Der Signalprozessor 42 führt die bestimmungsgemäße Funktion des Hörimplantates aus. Dazu gehören eine Audiosignalverarbeitung für die Rehabilitation einer Hörstörung und gegebenenfalls auch eine Signalgenerierung im Fall eines Systems mit zusätzlicher Tinnitusmaskierer- oder Noiser-Funktion. Weiterhin enthält der digitale Signalprozessor 42 Softwaremodule, die eine duale Wandler- und Reizelektrodenansteuerung so vornehmen, daß die spektralen, zeitlichen, amplituden- und phasenbezogenen Wandler- bzw. Reizelektrodensignaleigenschaften so konfiguriert sind, daß bei dem betreffenden Patienten der optimale Hörerfolg erzielt wird. Von besonderem Vorteil kann es sein, daß mit dem intracochleären, "dualen" Wandlerarray in einen flüssigkeitsgefüllten Raum des Innenohres (scala tympani oder scala vestibuli) nicht nur einer, sondern mehrere elektromechanische Wandler direkt implantiert werden, die die flüssigkeitsgefüllten Innenohrräume an örtlich verschiedenen Bereichen entlang der Basilarmembran vibratorisch stimulieren und so zu einem Höreindruck führen. Dabei werden die einzelnen elektromechanischen Wandler vorteilhaft so von einem elektronischen Vorverarbeitungssystem angesteuert, daß durch jeweilige Wahl des spektralen Übertragungsbereiches je Wandler, der vibratorischen Amplitude und der Phasenlage der Wandler zueinander im aktorischen Gesamtergebnis der Innenohrstimulation eine Wanderwellenausbildung auf der Basilarmembran entsteht, die bei dem jeweiligen externen Schallereignis der Wanderwellenform möglichst nahekommt, die sich bei nicht geschädigtem cochleären Verstärker und damit intakten äußeren Haarzellen ergeben würde.

Diese Softwaremodule können statisch und dynamisch ausgelegt sein. Unter statischer Auslegung soll dabei verstanden werden, daß die Softwaremodule aufgrund wissenschaftlicher Erkenntnisse einmalig im Programmspeicher des Signalprozessors 42 abgelegt werden und unverändert bleiben. Dynamisch bedeutet, daß diese Softwaremodule "lernfähig" sind, um dem angestrebten optimalen Hörergebnis zeitlich iterativ möglichst nahe zu kommen. Dies bedeutet, daß die Softwaremodule adaptiv ausgelegt sein können und die Parameteranpassung durch "Training" durch den Implantatträger und gegebenenfalls weitere Hilfsmittel, wie Rehabilitationsprogramme, vorgenommen wird. Weiterhin kann ein Softwaremodul enthalten sein, welches eine möglichst optimale Hörversorgung auf der Basis eines lernfähigen neuronalen Netzwerkes approximiert. Das Training dieses neuronalen Netzes kann wieder durch den Implantatträger und/oder unter Zuhilfenahme weiterer externer Hilfsmittel erfolgen.

Eine Methode zur möglichst optimalen Simulation des "gesunden" cochleären Verstärkers durch mehrkanalige elektromechanische Stimulation durch das elektromechanische Wandlerarray kann die softwaremäßige Implementierung des Prinzips der "Time-Reversed Acoustics" (TRA) sein (Fink, M.: "Time-Reversed Acoustics", Scientific American 281:5 (1999), p. 67-73). Die Ansteuerung der elektromechanischen Wandlerelemente 14 erfolgt mittels TRA so, daß örtlich begrenzte Bereiche der Cochlea mechanisch stimuliert werden. Während in herkömmlichen Anwendungen von TRA die Registrierung des verteilten Schallereignisses und die Aussendung des time-reversed Signals im selben Präparat erfolgen, werden diese beiden Schritte im vorliegenden Fall getrennt. Die verteilten Ereignisse können zum Beispiel in einem adäquaten Tiermodell intracochleär ermittelt werden; anschließend werden die time-reversed Stimuli bei der vorliegenden Applikation eines Hörsystems für den Menschen, gegebenenfalls unter Parameteranpassung an die veränderte Geometrie der menschlichen Cochlea, appliziert.

Damit die beschriebenen, software-basierten Algorithmen zur möglichst optimalen dualen Stimulation des geschädigten Gehörs insbesondere in einem Vollimplantat auch postoperativ implementiert werden können, enthält das System nach Fig. 5 einen weiteren Mikroprozessorbaustein, zum Beispiel einen Mikrokontroller (µC) 44 mit zugehörigen Speichern S₃, S₄ und S₅. Bei dem Speicher S₃ handelt es sich um einen wiederholt beschreibbaren Speicher, in welchem ein Arbeitsprogramm für den Microcontroller 44 abgelegt ist. In den Speicherbereichen S₄ und S₅ können insbesondere die Betriebsoftwareanteile des Implantatmanagementsystems abgelegt sein (zum Beispiel Verwaltungsüberwachungs- und Telemetriefunktionen). In den Speichern S₁ und/oder S₂ und/oder S₄ und/oder S₅ können auch von außen veränderliche, patientenspezifische wie zum Beispiel audiologische Anpaßparameter, abgelegt sein.

Der Mikrokontroller 44 kommuniziert einerseits über einen bidirektionalen Datenbus 45 und ein Telemetriesystem (TS) 46 drahtlos (beispielsweise mittels induktiver Kopplung) durch die bei 47 angedeutete geschlossene Haut mit einem externen Programmiersystem (PS) 48. Das Programmiersystem 48 kann ein PC-basiertes System mit entsprechender Programmier-, Bearbeitungs-, Darstellungs- und Verwaltungssoftware sein. Über diese Telemetrieschnittstelle wird die zu verändernde beziehungsweise ganz auszutauschende Betriebssoftware des Implantatsystems übertragen und zunächst in dem Speicherbereich S₄ und/oder S₅ des Mikrokontrollers 44 zwischengespeichert. So kann zum Beispiel eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software in die Programmspeicherbereiche S₁ und S₂ des digitalen Signalprozessors 42 über einen Datenbus 50 übertragen werden. Ferner kann auch das Arbeitsprogramm für den Mikrokontroller 44 über die Telemetrieschnittstelle ganz oder teilweise mit Hilfe der externen Einheit 48 geändert oder ausgetauscht werden.

Andererseits steuert der Mikrokontroller **44** implantatintern über den bidirektionalen Datenbus 50 den Signalprozessor **42,** die A/D-Konverter 41 der Sensor-Vorverarbeitung und die D/A-Konverter 43 zur Ansteuerung der elektromechanischen Wandler 14 und zur Ansteuerung von den Cochlear-Implant-Elektroden 11 beziehungsweise den Haarzellen-Reizelektroden 21 zugeordneten Stromquellen. Über den Datenbus 50 werden auch Programmteile oder ganze Softwaremodule zwischen Außenwelt, Mikrokontroller 44 und Signalprozessor 42 übermittelt.

Das Implantatsystem enthält bei vollimplantierter Ausführungsform auch eine primäre oder sekundäre Batteriezelle 52, die die einzelnen Module mit elektrischer Betriebsenergie versorgt.

Werden anstelle der elektromechanischen Wandler 14 in Verbindung mit der Schaltungsanordnung der Fig. 5 Haarzellen-Reizelektroden 21 zur mittelbaren mechanischen Innenohrstimulation vorgesehen, versteht es sich, daß diesen Elektroden 21 vorzugsweise Stromquellen entsprechend den in Fig. 5 dargestellten Stromquellen 51 für die Cochlea-Implant-Elektroden 11 vorgeschaltet werden.

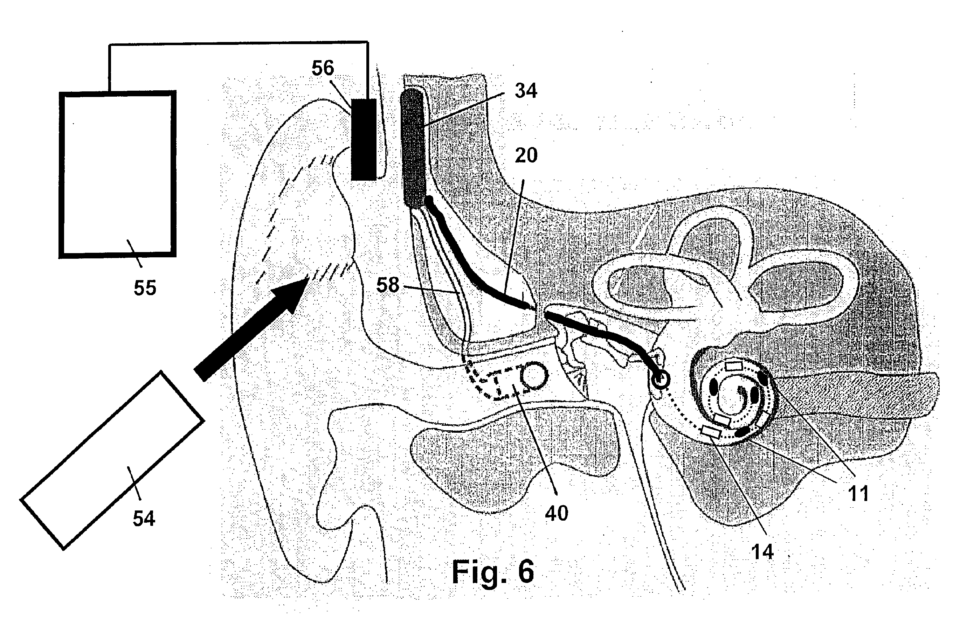

Fig. 6 zeigt schematisch den Aufbau eines vollständig implantierbaren Hörsystems mit einem intracochleären Stimulationsarray 10 gemäß Fig. 1, einem signalverarbeitenden Elektronikmodul 34 gemäß Fig. 5 und einem implantierbaren Mikrofon 40. Es können auch mehrere Mikrofone zur Aufnahme des Schallsignals und dessen Umwandlung in entsprechende elektrische Signale vorgesehen sein. Eine drahtlose Fernbedienung 54 dient der Steuerung der Implantatfunktionen durch den Implantatträger. Ferner ist ein Ladesystem mit einem Ladegerät 55 zum drahtlosen transkutanen Nachladen einer im Implantat befindlichen sekundären Batterie zur Energieversorgung des Hörsystems, beispielsweise der Batterie 52 in Fig. 5, vorgesehen. Es versteht sich, daß bei diesem Hörsystem anstelle des Stimulationsarray 10 gemäß Fig. 1 auch das Stimulationsarray 22 gemäß Fig. 2 oder eine Anordnung mit einer Kombination von Cochlea-Implant-Elektroden 11 und Haarzellen-Reizelektroden 21 gemäß Fig. 4 verwendet werden kann.

Das Mikrofon 40 kann vorteilhaft in der aus EP-A-0 831 673 bekannten Weise aufgebaut und mit einer Mikrofonkapsel, die in einem Gehäuse allseitig hermetisch dicht untergebracht ist, sowie mit einer elektrischen Durchführungsanordnung zum Durchführen mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind, wobei der eine Schenkel die Mikrofonkapsel aufnimmt und mit einer Schalleintrittsmembran versehen ist, wobei der andere Schenkel die elektrische Durchführungsanordnung enthält und gegenüber der Ebene der Schalleintrittsmembran zurückversetzt ist, und wobei die Geometrie des Mikrofongehäuses so gewählt ist, daß bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des Mikrofons 40 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteile versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Zu dem Ladesystem gehört auch eine an den Ausgang des Ladegerätes 55 angeschlossene Ladespule 56, die vorzugsweise in der aus US-A-5 279 292 bekannten Art Teil eines Sende-Serienresonanzkreises bildet, der mit einem nicht veranschaulichten Empfangs-Serienresonanzkreis induktiv gekoppelt werden kann. Der Empfangs-Serienresonanzkreis kann bei der Ausführungsform gemäß Fig. 5 Teil des Elektronikmoduls 34 sein und entsprechend US-A-5 279 292 eine Konstantstromquelle für die Batterie 52 (Fig. 5) bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 34 ist bei der Anordnung nach Fig. 6 über eine Mikrofonleitung 58 an das Mikrofon 40 und über die Wandlerarray-Zuleitung 20 an das intracochleäre Stimulationsarray 10 angeschlossen.

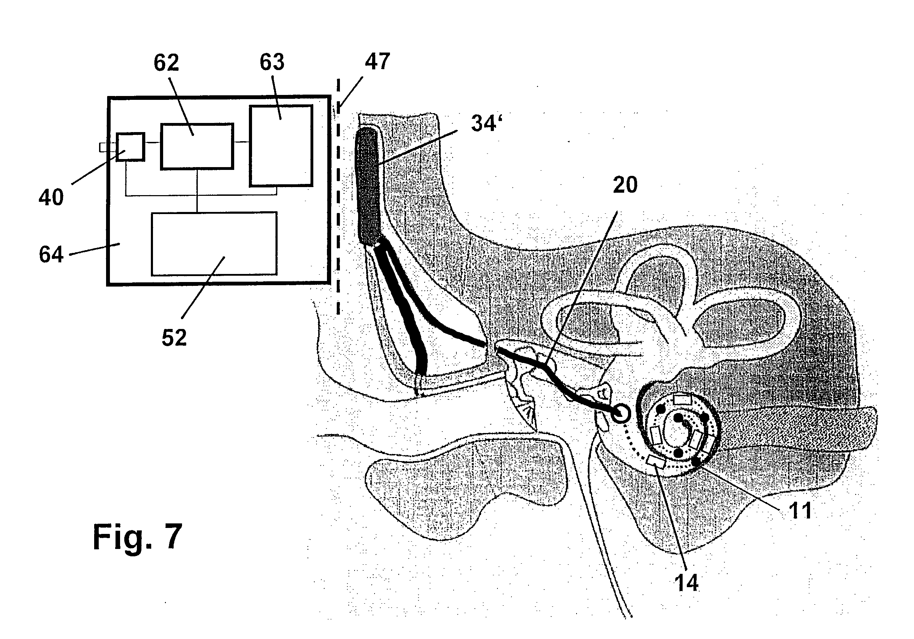

Fig. 7 zeigt schematisch den Aufbau eines teilimplantierbaren Hörsystems mit einem intracochleären Stimulationsarray 10 gemäß Fig. 1. Bei diesem teilimplantierbaren System sind ein Mikrofon 40, ein Elektronikmodul 62 für eine elektronische Signalverarbeitung weitestgehend entsprechend Fig. 5 (aber ohne das Telemetriesystem 46), die Energieversorgung 52 sowie eine Modulator/Sender-Einheit 63 in einem extern am Körper, vorzugsweise am Kopf über dem Implantat, zu tragenden externen Modul 64 enthalten. Das Implantat ist wie bei bekannten Teilimplantaten energetisch passiv. Sein Elektronikmodul 34' (ohne Batterie 52) empfängt seine Betriebsenergie und Wandlersteuerdaten über die Modulator/Sender-Einheit 63 im externen Teil 64. Es versteht sich, daß bei einem solchen teilimplantierbaren Hörsystem auch das Stimulationsarray gemäß Fig. 2 oder Reizelektroden gemäß Fig. 4 vorgesehen sein können und daß auch eine binaurale Anwendung ähnlich den nachstehend erläuterten Ausführungsbeispielen möglich ist.

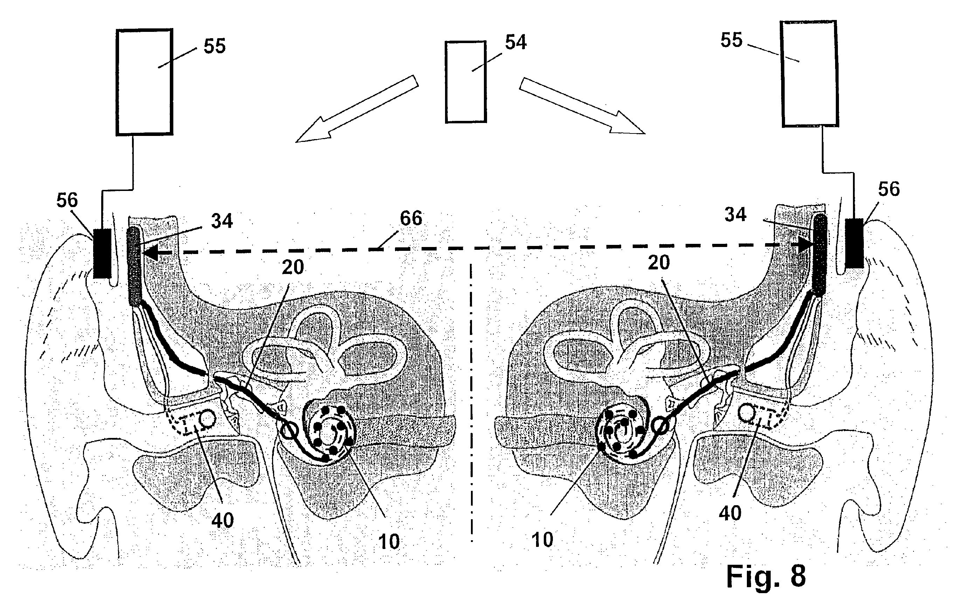

Fig. 8 zeigt eine binaurale Anwendung eines vollimplantierbaren Hörimplantates der anhand der Figuren 1 bis 6 erläuterten Art. Dabei sind, ebenso wie in den nachfolgenden Figuren, die intracochleären "dualen" Stimulationsarrays 10 (beziehungsweise 22) ohne Details nur schematisch angedeutet. Die Signalverarbeitungsmodule 34 der beiden Systeme kommunizieren über eine drahtgebundene implantierbare Leitungsverbindung 66 so miteinander, daß eine optimale binaurale sensorische Signalverarbeitung und Stimulationsarray-Ansteuerung in beiden implantierten Innenohren erreicht wird. Weiterhin sind auch hier transkutane Ladevorrichtungen 55, 56 für den Fall implantatseitiger sekundärer Energiespeicherelemente (Batterien 52) vorgesehen sowie eine beide Elektronikmodule 34 synchron steuernde drahtlose Fernbedienung 54 zur Anwendung durch den Implantatträger.

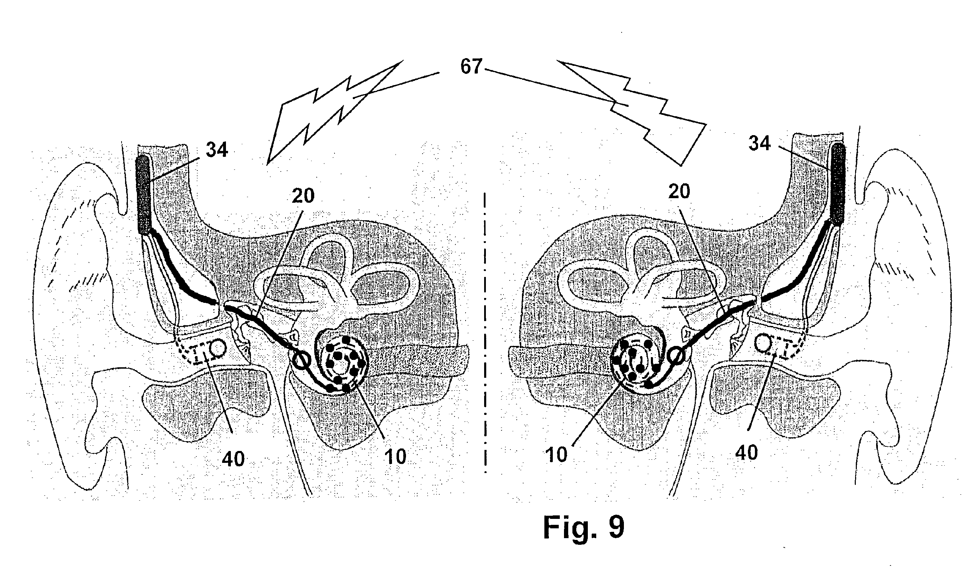

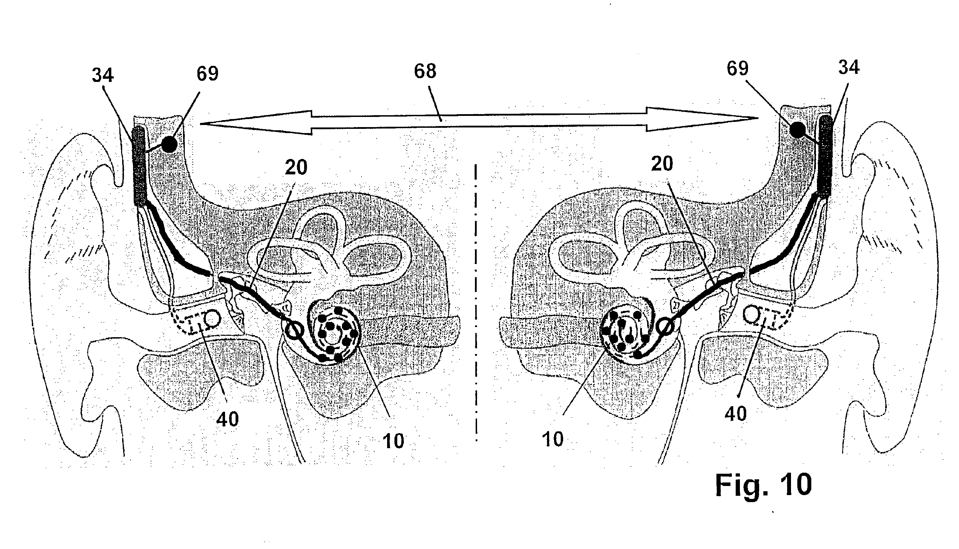

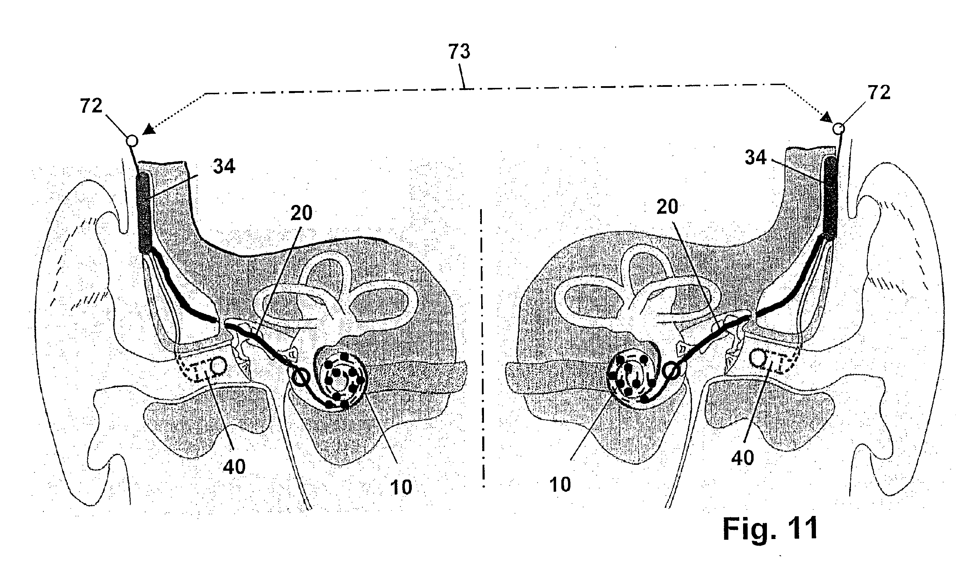

Fig. 9 zeigt die binaurale Anwendung eines Hörimplantates, bei der die Signalverarbeitungsmodule 34 über eine drahtlose Verbindung (zum Beispiel eine bei 67 angedeutete bidirektionale Hochfrequenzstrecke) so miteinander kommunizieren, daß eine optimale binaurale sensorische Signalverarbeitung und Stimulationsarray-Ansteuerung in beiden implantierten Innenohren erreicht wird. Transkutane Ladevorrichtungen 55, 56 für den Fall implantatseitiger sekundärer Energiespeicherelemente (Batterien 52) sowie eine beide Elektronikmodule 34 synchron steuernde drahtlose Fernbedienung 54 zur Anwendung durch den Implantatträger sind auch hier vorgesehen aber ebenso wie bei den beiden nachstehend beschriebenen und in den Figuren 10 und 11 veranschaulichten Ausführungsbeispielen nicht dargestellt.

Die binaurale Ausbildung des Hörimplantates gemäß Fig. 10 unterscheidet sich von denjenigen der Fig. 9 nur dadurch, daß für die drahtlose Kommunikation zwischen den Signalverarbeitungsmodulen 34 beider Systemeinheiten eine körperschallgekoppelte Ultraschallstrecke 68 mit Ultraschall-Kopplern 69 vorgesehen ist. Dabei sind die zum Beispiel digitalen, bidirektionalen Informationen auf einen Träger im Ultraschallbereich vorzugsweise amplituden- oder frequenzmoduliert. Die Ultraschall-Koppler 69 können, wie in Fig. 10 dargestellt, von dem Elektronikmodul 34 örtlich getrennte und über elektrische Leitungen angeschlossene Ultraschallempfänger und -sender sein, die vorzugsweise im Mastoidbereich an den Schädelknochen fest angekoppelt sind. Die Ultraschall-Koppler können aber auch in den Elektronikmodulen 34 integriert sein (nicht dargestellt), wenn die Elektronikmodule so im Mastoidbereich implantiert werden, daß eine Ultraschall-Körperübertragung durch den Schädelknochen stattfinden kann.

Eine weiter abgewandelte Ausführungsform eines binaural ausgebildeten Hörimplantates ist in Fig. 11 dargestellt. Bei dieser Ausführungsform werden abweichend von den Ausführungsbeispielen der Figuren 8 bis 10 die zum Beispiel digitalen, bidirektionalen Informationen implantatseitig auf einen Träger vorzugsweise amplituden- oder frequenzmoduliert und an implantierte Elektroden 72 angelegt, die Teil einer durch Körpergewebe des Implantatträgers führenden Datenübertragungsstrecke 73 sind. Es wird so ein modulierter Gewebestrom erhalten, der in an sich bekannter Weise (DE-A-38 31 809) für die gewünschte Kommunikation zwischen den Signalverarbeitungsmodulen 34 beider Systemeinheiten sorgt.

Es versteht sich, daß auch ein teilimplantierbares System binaural appliziert werden kann und daß dann für eine Kommunikation zwischen den beiden Systemeinheiten vorzugsweise entsprechend den in den Figuren 8 bis 11 veranschaulichen Ausführungsbeispielen von binauralen Anwendungen vollimplantierbarer Systeme gesorgt werden kann.

## Patentansprüche

1. Mindestens teilweise implantierbares System zur Rehabilitation einer Hörstörung, das mindestens einen Sensor (Mikrofon) (40) zur Aufnahme des Schallsignals und dessen Umwandlung in entsprechende elektrische Signale, eine elektronische Signalverarbeitungseinheit (34, 34') zur Audiosignalverarbeitung und -verstärkung, eine elektrische Energieversorgungseinheit (52), welche einzelne Komponenten des Systems mit Strom versorgt, sowie eine ausgangsseitige aktorische Stimulationsanordnung umfasst, **dadurch gekennzeichnet, daß** als aktorische Stimulationsanordnung eine duale intracochleäre Anordnung (10) vorgesehen ist, die in Kombination eine Stimulatoranordnung mit mindestens einem Stimulatorelement (14 und/oder 21) zur mindestens mittelbaren mechanischen Stimulation des Innenohres und eine elektrisch wirkende Reizelektrodenanordnung mit mindestens einer Cochlea-Implant-Elektrode (11) zur elektrischen Stimulation des Innenohres aufweist.

2. System nach Anspruch 1, **dadurch gekennzeichnet, daß** die Stimulatoranordnung mindestens einen intracochleären elektromechanischen Wandler (14) zur unmittelbaren mechanischen Stimulation des Innenohres aufweist.

3. System nach Anspruch 1, **dadurch gekennzeichnet, daß** die Stimulatoranordnung mindestens eine intracochleäre Haarzellen-Reizelektrode (21) zur mittelbaren mechanischen Stimulation des Innenohres durch elektrische Reizung von äußeren Haarzellen aufweist.

4. System nach einem der vorhergehenden Ansprüche, **gekennzeichnet durch** ein intracochleäres Array mit mehreren Stimulatorelementen (14 und/oder 21) zur unmittelbaren und/oder mittelbaren mechanischen Stimulation des Innenohres.

5. System nach Ansprüchen 3 und 4, **dadurch gekennzeichnet, daß** die zur mittelbaren mechanischen Stimulation des Innenohres bestimmten Haarzellen-Reizelektroden (21) so ausgebildet sind, daß sie im implantierten Zustand in unmittelbarer Nähe der äußeren Haarzellen zu liegen kommen.

6. System nach einem der vorhergehenden Ansprüche, **dadurch gekennzeichnet, daß** die zur elektrischen Stimulation des Innenohres bestimmte(n) Cochlea-Implant-Elektrode(n) (11) so in einen Träger (13) eingebettet oder an dem Träger fixiert ist (sind), daß im implantierten Zustand ein Oberflächenanteil jeder Cochlea-Implant-Elektrode (11) in direktem galvanischem Kontakt zu der lymphatischen Flüssigkeit des Innenohres oder direkt zu einer der zu reizenden neuronalen Strukturen steht.

7. System nach Anspruch 2, **gekennzeichnet durch** einen gemeinsamen Träger (13) für die Cochlea-Implant-Elektrode(n) (11) und den oder die elektromechanischen Wandler (14).

8. System nach Anspruch 7, **dadurch gekennzeichnet, daß** elektromechanische Wandler (14) und Cochlea-Implant-Elektroden (11) entlang dem Träger (13) einander abwechselnd angeordnet sind.

9. System nach Anspruch 2, **dadurch gekennzeichnet, daß** für die Cochlea-Implant-Elektrode(n) (11) und den oder die elektromechanischen Wandler (14) separate Träger (23, 24) vorgesehen sind.

10. System nach Anspruch 9, **dadurch gekennzeichnet, daß** der Träger (24) für die Cochlea-Implant-Elektrode(n) so angeordnet ist, daß er im implantierten Zustand bezüglich der Cochlea nach innen orientiert ist.

11. System nach einem der Ansprüche 7 bis 10, **dadurch gekennzeichnet, daß** der Gesamtdurchmesser der dualen intracochleären Anordnung (10) im Bereich von 0,4 mm (apikaler Bereich) bis 2,0 mm (basaler Bereich) liegt.

12. System nach einem der Ansprüche 7 bis 11, **dadurch gekennzeichnet, daß** die Gesamtlänge der dualen intracochleären Anordnung (10) zwischen 5 mm und 50 mm liegt.

13. System nach einem der Ansprüche 7 bis 12, **dadurch gekennzeichnet, daß** der oder die Träger (13; 23, 24) aus einem biokompatiblen und im Innenohr biostabilen Material, vorzugsweise einem Polymer, insbesondere einem Silikon, besteht beziehungsweise bestehen.

14. System nach einem der Ansprüche 7 bis 13, **dadurch gekennzeichnet, daß** die einzelnen elektromechanischen Wandler (14) so in den zugehörigen Träger (13, 23) eingebettet sind, daß sie von einer dünnen Schicht des Trägermaterials vollständig umgeben sind.

15. System nach nach einem der Ansprüche 7 bis 14, **dadurch gekennzeichnet, daß** zwischen den einzelnen elektromechanischen Wandlern (14) mechanische Dämpfungselemente (11, 25) in den Träger (13, 23) eingebettet sind, die die mechanische Wellenausbreitung innerhalb des Trägers zu benachbarten Wandlern minimieren.

16. System nach Anspruch 15, **dadurch gekennzeichnet, daß** das Material der Dämpfungselemente (11, 25) bei ähnlicher Querschnittsgeometrie wie die des Trägers (13, 23) so gewählt ist, daß zur Erzielung hoher Dämpfungswerte ein großer mechanischer Impedanzunterschied zu dem Trägermaterial besteht.

17. System nach Ansprüchen 8 und 16 **dadurch gekennzeichnet, daß** die Dämpfungselemente von Cochlea-Implant-Elektroden (11) gebildet sind.

18. System nach einem der Ansprüche 2 und 6 bis 17, **dadurch gekennzeichnet, daß** der beziehungsweise die elektromechanische(n) Wandler (14) als nach dem elektromagnetischen, elektrodynamischen, piezoelektrischen, magnetostriktiven oder dielektrischen (kapazitiven) Prinzip arbeitender Wandler ausgebildet ist (sind).

19. System nach Anspruch 18, **dadurch gekennzeichnet, daß** der beziehungsweise die elektromechanische(n) Wandler (14) bei Anwendung des piezoelektrischen Wandlerprinzips unter Verwendung von PZT-Keramik (Blei-Zirkonat-Titanat) oder PVDF (Polyvinylidenfluorid) aufgebaut ist (sind).

20. System nach einem der Ansprüche 2 und 6 bis 19, **dadurch gekennzeichnet, daß** der beziehungsweise die elektromechanische(n) Wandler (14), vorzugsweise mit Ausnützung von geometrischen Gestalttransformationen, insbesondere des Bimorph-Prinzips, des Unimorph-Prinzips oder des Heteromorph-Prinzips mit passiven Materialpartnern, so ausgeführt ist (sind), daß er (sie) bei gegebener Wandlerspannung eine maximale Auslenkung bei minimaler elektrischer Leistungsaufnahme erzeugt (erzeugen).

21. System nach einem der Ansprüche 4 bis 20, **dadurch gekennzeichnet, daß** mehrere Stimulatorelemente (14 und/oder 21) zur mindestens mittelbaren mechanischen Stimulation des Innenohres äquidistant oder in logarithmischen Distanzen - entsprechend der tonotopen Frequenz-Orts-Zuordnung längs der Basilarmembran des Innenohres - verteilt angeordnet sind.

22. System nach Anspruch 21, **dadurch gekennzeichnet, daß** bei tonotoper Anordnung der Stimulatorelemente (14, 21) zur mindestens mittelbaren mechanischen Stimulation des Innenohres 20 bis 24 Stimulatorelemente (14) oder Gruppen von Stimulatorelementen (21) gemäß den psychoakustischen Frequenzgruppen vorgesehen sind.

23. System nach einem der Ansprüche 2 und 6 bis 22, **dadurch gekennzeichnet, daß** der beziehungsweise die elektromechanische(n) Wandler (14) einen Übertragungsbereich von etwa 100 Hz bis etwa 10 kHz hat (haben).

24. System nach einem der Ansprüche 2 und 6 bis 23, **dadurch gekennzeichnet, daß** der beziehungsweise die elektromechanische(n) Wandler (14) so abgestimmt ist (sind), daß seine (ihre) erste mechanische Resonsanzfrequenz am oberen Ende des angestrebten Übertragungsfrequenzbereiches, insbesondere bei etwa 8 kHz bis etwa 10 kHz, liegt.

25. System nach einem der Ansprüche2 und 6 bis 24, **dadurch gekennzeichnet, daß** der beziehungsweise die elektromechanische(n) (14) hermetisch dicht aufgebaut ist (sind).

26. System nach einem der vorhergehenden Ansprüche, **dadurch gekennzeichnet, daß** die Cochlea-Implant-Elektrode(n) (11) und/oder die Haarzellen-Reizelektrode(n) (21) aus Platin, aus Platin-Iridium-Legierungen, vorzugsweise mit etwa 90 % Pt und etwa 10 % Ir, oder aus Gold, Goldlegierungen, Tantal, Tantallegierungen, Niob, Nioblegierungen oder Edelstahl hergestellt sind.

27. System nach einem der vorhergehenden Ansprüche, **dadurch gekennzeichnet, daß** die Signalverarbeitungseinheit (34, 34', 62) eine Vorverarbeitungsanordnung (41) zum Vorverstärken und/oder Filtern sowie zum Analog-Digital- (A/D-) Wandeln der Schallsensorsignale aufweist.

28. System nach Anspruch 27, **dadurch gekennzeichnet, daß** die Vorverarbeitungsanordnung einen Antialiasing-Filter umfaßt.

29. System nach einem der vorhergehenden Ansprüche, **dadurch gekennzeichnet, daß** bei Vorhandensein mehrerer Schallsensoren (40) jedem der Schallsensoren ein Analog-Digital-Wandler (41) nachgeschaltet ist.

30. System nach einem der vorhergehenden Ansprüche, **dadurch gekennzeichnet, daß** die Signalverarbeitungseinheit (34, 34', 62) Softwaremodule enthält, die parallel zum Hörgerätebetrieb die Maskierung eines Tinnitus ermöglichen.

31. System nach einem der vorhergehenden Ansprüche, **dadurch gekennzeichnet, daß** die Signalverarbeitungseinheit (34, 34', 62) einen digitalen Signalprozessor (42) zum Verarbeiten der A/D-gewandelten und gegebenenfalls mittels der Vorverarbeitungsanordnung (41) vorverarbeiteten Schallsensorsignale und/oder zum Generieren von digitalen Signalen für eine Tinnitusmaskierung aufweist.

32. System nach einem der vorhergehenden Ansprüche, **gekennzeichnet durch** mindestens einen der aktorischen Stimulationsanordnung (10, 22) vorgeschalteten Digital-Analog-Wandler (43).

33. System nach Anspruch 32, **dadurch gekennzeichnet, daß** dem beziehungsweise den Stimulatorelement(en) (14, 21) zur mindestens mittelbaren mechanischen Stimulation des Innenohres und der beziehungsweise den Cochlea-Implant-Elektrode(n) (11) zur elektrischen Stimulation des Innenohres jeweils ein eigener Digital-Analog-Wandler (43) vorgeschaltet ist.

34. System nach einem der vorhergehenden Ansprüche, **dadurch gekennzeichnet, daß** die Signalverarbeitungselektronik (42, 44) Softwaremodule enthält, die die Ansteuerung der Stimulatorelemente (14 und/oder 21) zur mindestens mittelbaren mechanischen Stimulation des Innenohres so vornehmen, daß die spektralen, zeitlichen, amplituden- und phasenbezogenen Eigenschaften der die Stimulatorelemente ansteuernden Signale so bemessen sind, daß auf der Basilarmembran des geschädigten Innenohres eine Wanderwelle erzeugt wird, die der eines gesunden Gehörs möglichst nahe kommt.

35. System nach Anspruch 34, **dadurch gekennzeichnet, daß** die Softwaremodule statisch in der Weise ausgelegt sind, daß sie aufgrund wissenschaftlicher Erkenntnisse einmalig in einem Programmspeicher (S₁, S₂) des digitalen Signalprozessors (42) abgelegt werden und unverändert bleiben.

36. System nach einem der vorhergehenden Ansprüche, **gekennzeichnet durch** eine vorzugsweise PC-basierte, drahtlose Telemetrieeinrichtung (46) zur Übertragung von Daten zwischen dem implantierten Teil (34) des Systems und einer externen Einheit, insbesondere einem externen Programmiersystem (48).

37. System nach Ansprüchen 34 und 36, **dadurch gekennzeichnet, daß** die Softwaremodule dynamisch (lernfähig) ausgelegt sind.

38. System nach Anspruch 37, **dadurch gekennzeichnet, daß** die Softwaremodule für eine Parameteranpassung durch "Training" durch den Implantatträger und/oder unter Zuhilfenahme weiterer externer Hilfsmittel adaptiv ausgelegt sind.

39. System nach einem der vorhergehenden Ansprüche, **dadurch gekennzeichnet, daß** die Signalverarbeitungselektronik (42, 44) ein Softwaremodul zum Approximieren einer möglichst optimalen Simulation eines "gesunden" cochleären Verstärkers auf der Basis eines lernfähigen neuronalen Netzwerkes enthält.

40. System nach Anspruch 39, **dadurch gekennzeichnet, daß** das neuronale Netzwerk für ein "Training" durch den Implantatträger und/oder für ein Lernen unter Zuhilfenahme externer Hilfsmittel ausgelegt ist.

41. System nach Anspruch 39 oder 40, **dadurch gekennzeichnet, daß** in dem neuronalen Netzwerk zur Simulation eines "gesunden" cochleären Verstärkers das Prinzips der "Time-Reversed Acoustics" (TRA) implementiert ist und die Ansteuerung der Stimulatorelemente (14, 21) zur mechanischen Stimulation des Innenohres mittels TRA so erfolgt, daß örtlich begrenzte Bereiche der Cochlea mechanisch stimuliert werden.

42. System nach einem der Ansprüche 36 bis 41, **dadurch gekennzeichnet, daß** dem Signalprozessor (42) zur Aufnahme und Wiedergabe eines Betriebsprogramms eine wiederholt beschreibbare, implantierbare Speicheranordnung (S₁, S₂) zugeordnet ist, und mindestens Teile des Betriebsprogramms durch von der externen Einheit (48) über die Telemetrieeinrichtung (46) übermittelte Daten geändert oder ausgetauscht werden können.

43. System nach Anspruch 42, **dadurch gekennzeichnet, daß** ferner eine Zwischenspeicheranordnung (S₄, S₅) vorgesehen ist, in welcher von der externen Einheit (48) über die Telemetrieeinrichtung (46) übermittelte Daten vor dem Weiterleiten an den Signalprozessor (42) zwischengespeichert werden können.

44. System nach Anspruch 43, **dadurch gekennzeichnet, daß** ferner eine Überprüfungslogik (44) vorgesehen ist, um in der Zwischenspeicheranordnung (S₄, S₅) gespeicherte Daten vor dem Weiterleiten an den Signalprozessor (42) einer Überprüfung zu unterziehen.

45. System nach einem der Ansprüche 31 bis 44, **gekennzeichnet durch** einen Mikroprozessorbaustein (44), insbesondere einen Microcontroller, zum implantatinternen Steuern der A/D-Wandler (41) und/oder der D/A-Wandler (43) und/oder des Signalprozessors (42) über einen Datenbus (50).

46. System nach den Ansprüchen 44 und 45, **dadurch gekennzeichnet, daß** die Überprüfungslogik und die Zwischenspeicheranordnung (S₄, S₅) in dem Mikroprozessorbaustein (44) implementiert sind.

47. System nach Anspruch 45 oder 46, **dadurch gekennzeichnet, daß** über den Datenbus (50) und die Telemetrieeinrichtung (46) auch Programmteile oder ganze Softwaremodule zwischen der Außenwelt, dem Mikroprozessorbaustein (44) und dem Signalprozessor (42) übermittelbar sind.

48. System nach einem der Ansprüche 45 bis 47, **dadurch gekennzeichnet, daß** dem Mikroprozessorbaustein (44) eine implantierbare Speicheranordnung (S₃) zum Speichern eines Arbeitsprogramms für den Mikroprozessorbaustein zugeordnet ist, und mindestens Teile des Arbeitsprogramms für den Mikroprozessorbaustein durch von der externen Einheit (48) über die Telemetrieeinrichtung (46) übermittelte Daten geändert oder ausgetauscht werden können.

49. System nach einem der Ansprüche 42 bis 48, **dadurch gekennzeichnet, daß** mindestens zwei Speicherbereiche (S₁, S₂) zur Aufnahme und Wiedergabe mindestens des Betriebsprogramms des Signalprozessors (42) vorgesehen sind.

50. System nach einem der Ansprüche 44 bis 49, **dadurch gekennzeichnet, daß** die Zwischenspeicheranordnung mindestens zwei Speicherbereiche (S₄, S₅) zur Aufnahme und Wiedergabe von von der externen Einheit (48) über die Telemetrieeinrichtung (46) übermittelten Daten aufweist.

51. System nach einem der Ansprüche 31 bis 50, **dadurch gekennzeichnet, daß** dem Signalprozessor (42) ferner ein vorprogrammierter, nicht überschreibbarer Festspeicherbereich (S₀) zugeordnet ist.

52. System nach einem der Ansprüche 31 bis 51, **dadurch gekennzeichnet, daß** die Telemetrieeinrichtung (46) zur Übermittlung auch von Betriebsparametern zwischen dem implantierbaren Teil (34) des Systems und der externen Einheit (48) ausgelegt ist.

53. System nach einem der vorhergehenden Ansprüche, **dadurch gekennzeichnet, daß** es vollimplantierbar ausgebildet und mit mindestens einem implantierbaren Schallsensor (40) versehen ist, die elektrische Energieversorgungseinheit implantatseitig ein nachladbares elektrisches Speicherelement (52) aufweist und eine drahtlose, transkutane Ladevorrichtung (55, 56) zum Laden des Speicherelements vorgesehen ist.

54. System nach Anspruch 53, **gekennzeichnet durch** eine drahtlose Fernbedienung (54) zur Steuerung der Implantatfunktionen **durch** den Implantatträger.

55. System nach einem der Ansprüche 1 bis 52, **dadurch gekennzeichnet, daß** es teilimplantierbar ausgebildet ist, wobei mindestens ein Schallsensor (40), die elektronische Anordnung (62) zur Audiosignalverarbeitung und -verstärkung, die Energieversorgungseinheit (52) sowie eine Modulator/Sender-Einheit (63) in einem extern am Körper, vorzugsweise am Kopf über dem Implantat (34'), zu tragenden externen Modul (64) enthalten sind, sowie das Implantat energetisch passiv ist und seine Betriebsenergie und Steuerdaten für die mechanische und elektrische Innenohrstimulation über die Modulator/Sender-Einheit im externen Modul empfängt.

56. System nach einem der vorhergehenden Ansprüche, **dadurch gekennzeichnet, daß** es als binaurales System zur Rehabilitation einer Hörstörung beider Ohren ausgelegt ist, das zwei Systemeinheiten aufweist, die jeweils einem der beiden Ohren zugeordnet sind.

57. System nach Anspruch 56, **dadurch gekennzeichnet, daß** die beiden Systemeinheiten einander im wesentlichen gleich sind.

58. System nach Anspruch 56, **dadurch gekennzeichnet, daß** die eine Systemeinheit als Master-Einheit und die andere Systemeinheit als von der Master-Einheit gesteuerte Slave-Einheit ausgelegt ist.

59. System nach einem der Ansprüche 56 bis 58, **gekennzeichnet durch** eine drahtgebundene implantierbare Leitungsverbindung (66), über welche die Signalverarbeitungsmodule (34) so miteinander kommunizieren, daß in beiden Systemeinheiten eine optimierte binaurale Signalverarbeitung und Wandler-Array-Ansteuerung erreicht wird.

60. System nach einem der Ansprüche 56 bis 58, **gekennzeichnet durch** eine drahtlose Verbindung (67), vorzugsweise eine bidirektionale Hochfrequenzstrecke, über welche die Signalverarbeitungsmodule (34) so miteinander kommunizieren, daß in beiden Systemeinheiten eine optimierte binaurale Signalverarbeitung und Wandler-Array-Ansteuerung erreicht wird.

61. System nach einem der Ansprüche 56 bis 58, **dadurch gekennzeichnet, daß** die Signalverarbeitungsmodule (34) unter Verwendung von Ultraschall-Kopplern (69) über eine körperschallgekoppelte Ultraschallstrecke (68) so miteinander kommunizieren, daß in beiden Systemeinheiten eine optimierte binaurale Signalverarbeitung und Wandler-Array-Ansteuerung erreicht wird.

62. System nach einem der Ansprüche 56 bis 58, **dadurch gekennzeichnet, daß** den Signalverarbeitungsmodulen (34) implantierbare Elektroden (72) zugeordnet sind, die im implantierten Zustand Teil einer durch Körpergewebe des Implantatträgers führenden Datenübertragungsstrecke (73) für eine Kommunikation der Signalverarbeitungsmodule (34) der beiden Systemeinheiten sind.

63. System nach einem der vorhergehenden Ansprüche, **dadurch gekennzeichnet, daß** das beziehungsweise die Stimulatorelement(e) (14, 21) zur mechanischen Stimulation des Innenohres und die Reizelektrode(n) (11) zur elektrischen Stimulation des Innenohres wahlweise allein oder in Kombination simultan betreibbar sind.
